# EUROPÄISCHE PATENTSCHRIFT

(11) **EP 2 334 631 B1**
(45) Veröffentlichungstag und Bekanntmachung des Hinweises auf die Patenterteilung: **01.03.2017**
(21) Anmeldenummer: 09780561.8
(22) Anmeldetag: 14.07.2009
(51) Int. Cl.: C07C 51/25, C07C 51/43

(54) **VERFAHREN ZUR AUFTRENNUNG VON IN EINEM PRODUKTGASGEMISCH EINER PARTIELLEN HETEROGEN KATALYSIERTEN GASPHASENOXIDATION EINER C3-VORLÄUFERVERBINDING DER ACRYLSÄURE ALS HAUPTBESTANDTEIL ENTHALTENER ACRYLSÄURE UND ALS NEBENPRODUKT ENTHALTENEM GLYOXAL**
METHOD FOR SEPARATING ACRYLIC ACID, CONTAINED AS THE MAIN CONSTITUENT, AND GLYOXAL, CONTAINED AS A BY-PRODUCT, FROM A GASEOUS PRODUCT MIXTURE OF A C3 PRECURSOR COMPOUND OF ACRYLIC ACID
PROCEDE DE SEPARATION DE L'ACIDE ACRYLIQUE CONTENU COMME CONSTITUANT PRINCIPAL, ET DE GLYOXAL CONTENU COMME PRODUIT SECONDAIRE D'UN MELANGE DE GAZ DE PRODUIT D'UNE OXYDATION EN PHASE GAZEUSE PARTIELLE PAR CATALYSE HETEROGENE D'UN COMPOSE PRECURSEUR EN C3 D'ACIDE ACRYLIQUE

(30) Priorität: 28.07.2008 DE 102008040799; 28.07.2008 US 84109 P; 26.08.2008 US 91900 P; 26.08.2008 DE 102008041573
(43) Veröffentlichungstag der Anmeldung: 22.06.2011
(73) Patentinhaber: BASF SE, 67056 Ludwigshafen am Rhein (DE)
(72) Erfinder: BLUM, Till, Kuantan 25050 (MY); ZUROWSKI, Peter, 76829 Landau (DE); RISSEL, Steffen, 67281 Kirchheim (DE); HAREMZA, Sylke, 69151 Neckargemünd (DE); FRIESE, Thorsten, 68199 Mannheim (DE); JÄGER, Ulrich, 67354 Römerberg (DE); SCHLIEPHAKE, Volker, 67105 Schifferstadt (DE); MÜLLER-ENGEL, Klaus, Joachim, 76297 Stutensee (DE); HAMMON, Ulrich, 68163 Mannheim (DE); HEILEK, Jörg, 69245 Bammental (DE); MÜLLER, Imke, Britta, 69118 Heidelberg (DE)
(86) Internationale Anmeldenummer: PCT/EP2009/058984
(87) Internationale Veröffentlichungsnummer: WO 2010/012586

(56) Entgegenhaltungen:
- EP-A- 1 298 120
- EP-A1- 1 396 484
- EP-A1- 1 484 308
- EP-A1- 1 484 309
- WO-A-03/078378
- US-B1- 6 433 222
- US-B1- 6 448 439

## Beschreibung

Vorliegende Erfindung betrifft ein Verfahren zur Auftrennung von in einem Produktgasgemisch einer partiellen heterogen katalysierten Gasphasenoxidation einer C₃-Vorläuferverbindung der Acrylsäure als Hauptprodukt enthaltener Acrylsäure und als Nebenprodukt enthaltenem Glyoxal, bei dem man eine flüssige Phase P erzeugt, die zu wenigstens 90 % ihres Gewichtes aus Acrylsäure besteht und, bezogen auf die in ihr enthaltene molare Menge an Acrylsäure, wenigstens 200 mol.ppm Glyoxal enthält.

Acrylsäure ist ein bedeutendes Monomeres, das als solches und/oder in Form seiner Alkylester zur Erzeugung von im Hygienebereich eingesetzten Polymerisaten (z. B. Wasser superabsorbierenden) Verwendung findet (vgl. z. B. WO 02/055469 und WO 03/078378).

Die Herstellung von Acrylsäure kann z. B. durch heterogen katalysierte partielle Oxidation einer C₃-Vorläuferverbindung (z. B. Propylen, Propan, Acrolein, Propionaldehyd, Propionsäure, Propanol und/oder Glyzerin) in der Gasphase erfolgen (vgl. z. B. EP-A 990 636, US-A 5,198,578, EP-A 1 015 410, EP-A 1 484 303, EP-A 1 484 308, EP-A 1 484 309, US-A 2004/0242826 und WO 2006113ei336).

Grundsätzlich wird im Rahmen einer solchen heterogen katalysierten partiellen Gasphasenoxidation keine reine Acrylsäure, sondern lediglich ein Acrylsäure enthaltendes Produktgasgemisch erhalten, das neben Acrylsäure auch von Acrylsäure verschiedene Bestandteile enthält, von denen die Acrylsäure abgetrennt werden muss.

Sowohl die Art als auch der Mengenanteil der von Acrylsäure verschiedenen Bestandteile im Produktgasgemisch kann unter anderem durch die Reinheit der als Rohstoff eingesetzten C₃-Vorläuferverbindung sowie durch die Reaktionsbedingungen, bei denen die heterogen katalysierte partielle Gasphasenoxidation durchgeführt wird, beeinflusst werden (vgl. z. B. DE-A 101 31 297 und DE-A 10 2005 052 917).

Aus der EP-A 770 592 ist bekannt, dass das Produktgasgemisch einer solchen heterogen katalysierten partiellen Gasphasenoxidation unter anderem verschiedene Aldehyde als von Acrylsäure verschiedene Bestandteile enthalten kann. Aus der EP-A 770 592 ist ferner bekannt, dass geringste Mengen von in Acrylsäure verbliebenen aldehydischen Verunreinigungen die Eigenschaften der Acrylsäure signifikant beeinträchtigen können. So sollten gemäß der Lehre der EP-A 770 592 die einzelnen Aldehydanteile innerhalb einer Acrylsäure unterhalb von 1 ppm liegen, um im Rahmen der Verwendung einer solchen Acrylsäure in insbesondere radikalischen Polymerisationsreaktionen zur z. B. Herstellung von superabsorbierenden Polymeren oder von als Dispergiermittel für Ölbohrschlamm oder als Flockungsmittel wirksamen Polymeren die optimalen Produktqualitäten zu erzielen. Um diese Trenngrade zu erreichen, empfiehlt die EP-A 770 592 die Mitverwendung von Aldehydfängern. Deren zusätzlicher Bedarf macht jedoch gleichzeitig die Nachteiligkeit der in der EP-A 770 592 empfohlenen Verfahrensweise aus.

Aus der EP-A 1 298 120 ist bekannt, dass als ein mögliches Nebenprodukt einer heterogen katalysierten partiellen Gasphasenoxidation von C₃-Vorläufern zu Acrylsäure unter bestimmten Bedingungen auch der Aldehyd Glyoxal gebildet werden kann. Unter anderem auch deshalb, weil Glyoxal die unerwünschte radikalische Polymerisation von Acrylsäure fördert, empfiehlt die EP-A 1 298 120, die Acrylsäureherstellung so zu gestalten, dass die Glyoxalnebenproduktbildung minimiert wird (als mögliche Quelle für eine Glyoxalnebenproduktbildung im Rahmen einer heterogen katalysierten partiellen Gasphasenoxidation von C₃-Vorläufern der Acrylsäure hat die EP-A 1 298 120 unter anderem die im C₃-Vorläufer eventuell enthaltene C₂-Verunreinigung Ethylen ausgemacht).
Unter Mitverwendung von Umkehrosmosetrennverfahren sind gemäß der
EP-A 1 298 120 Produktgasgemische erhältlich, aus denen die Acrylsäure auch bei Kreisführung des Absorptionsmittels in flüssige Phasen überführbar ist, die weniger als 100 Gew.ppm Glyoxal enthalten. Aus derartigen flüssigen Phasen kann die Acrylsäure gemäß der Lehre der EP-A 1 298 120 nachfolgend mittels destillativer Trennverfahren vergleichsweise problemfrei abgetrennt werden. Nachteilig an dieser Verfahrensweise ist jedoch der Bedarf der Reversosmose, der die Raum-Zeit-Ausbeute mindert.

Die EP-A 1 396 484 offenbart eine von der in der EP-A 1 298 120 empfohlenen Vorgehensweise verschiedene Verfahrensweise zur Auftrennung von in einem Produktgasgemisch einer partiellen heterogen katalysierten Gasphasenoxidation einer C₃-Vorläuferverbindung der Acrylsäure als Hauptprodukt enthaltener Acrylsäure und als Nebenprodukt enthaltenem Glyoxal, die höhere Nebenproduktgehalte an Glyoxal toleriert (dies ist unter anderem insofern vorteilhaft, als es den Einsatz wirtschaftlich attraktiver, einen erhöhten Verunreinigungsgehalt aufweisender C₃-Vorläuferverbindungen zulässt).

Sie ist dadurch gekennzeichnet, dass die Acrylsäure und das Glyoxal aus dem Produktgasgemisch heraus zunächst in eine wässrige Lösung absorbiert werden. Aus dieser heraus wird das Wasser nachfolgend durch azeotrope Destillation (Rektifikation) abgetrennt. Dabei ist, um unerwünschte Polymerisatbildung weitgehend zurückzudrängen, so zu verfahren, dass die Rücklaufflüssigkeit bestimmte Wassergehalte aufweist und das Rücklaufverhältnis einen bestimmten Wert nicht unterschreitet. Des Weiteren sind bestimmte Temperaturverhältnisse einzuhalten.

Das Glyoxal reichert sich unter diesen Rahmenbedingungen in Form hoch siedender Hydrate gemeinsam mit der Acrylsäure im Kolonnensumpf an.

Aus der vorgenannten Sumpfflüssigkeit kann die Acrylsäure von den Glyoxalhydraten nachfolgend destillativ abgetrennt werden, wobei die Glyoxalhydrate die die unerwünschte radikalische Polymerisation der Acrylsäure fördernde Qualität von monomerem Glyoxal ganz offensichtlich nicht mehr oder allenfalls noch in signifikant vermindertem Umfang aufweisen.

Eigene Untersuchungen der Anmelderin haben ergeben, dass die Fähigkeit des Glyoxals, als Verunreinigung in Acrylsäure die Neigung der Acrylsäure zur unerwünschten radikalischen Polymerisation zu fördern, im Vergleich zu anderen möglichen Nebenproduktaldehyden einer heterogen katalysierten partiellen Gasphasenoxidation von C₃-Vorläuferverbindungen (wie z. B. Acetaldehyd, Formaldehyd, Propionaldehyd, Benzaldehyd, Butyraldehyd, Acrolein), bezogen auf gleiche molare Verunreinigungsgehalte, wesentlich ausgeprägter ist. Dies ist vermutlich darauf zurückzuführen, dass, wie sich als Ergebnis von quantenmechanischen Berechnungen von Dissoziationsenergien herausgestellt hat, der thermische Aufwand zur Spaltung von monomerem Glyoxal in zwei Formylradikale zum einen besonders gering ist, und die dabei resultierenden Formyl-Radikale zum anderen weitaus reaktiver als z. B. ein Wasserstoffradikal oder ein Methylradikal sind (CCSD(T)-Methode (Coupled Cluster including Single, Double (and Triple) excitations)).

Experimente im Zusammenhang mit Literaturstudien (z. B. L'actualité chimique, Mai 1982, Seiten 23 bis 31 und die in diesem Artikel zitierte Literatur) haben bestätigt, dass Hydrate des Glyoxals die vorgenannte ausgeprägte Polymerisationsförderwirkung von monomerem (molekularem) Glyoxal nicht mehr aufweisen.

Dabei bilden die Glyoxalhydrate zwei Gruppen von Hydrattypen.
Die erste Gruppe besteht aus dem monomeren Glyoxal-Monohydrat und aus dem monomeren Glyoxal-Dihydrat:

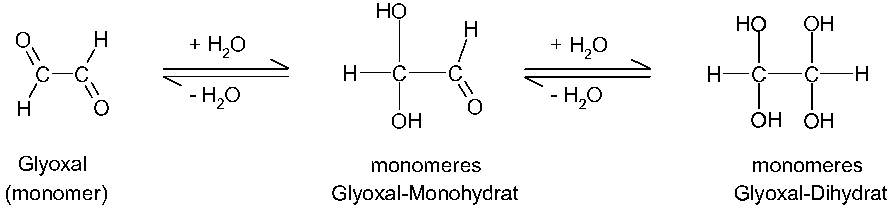

Beide vorgenannten Glyoxalhydrate bilden sich bereits unter vergleichsweise milden Bedingungen (niedere Temperaturen, beschränkte Wassergehalte sind ausreichend).

Allerdings ist sowohl die Bildungsreaktion des monomeren Glyoxal-Monohydrats als auch des monomeren Glyoxal-Dihydrats eine ausgesprochen reversible Reaktion. D. h., jedes der beiden vorgenannten Hydrate verfügt zwar nicht mehr über die ausgeprägte Polymerisationsförderwirkung von monomerem Glyoxal, jedoch kann sich aus beiden dieser Hydrate, z. B. bei mäßiger Temperaturerhöhung, wieder monomeres Glyoxal zurückbilden, welches dann in an sich bekannter Weise die unerwünschte radikalische Polymerisation der Acrylsäure zu begünstigen vermag. Im Folgenden sowie ganz generell in dieser Schrift (abgesehen von ihrer Präambel) soll der allein stehende Begriff "Glyoxal" daher stets die Gesamtmenge aus monomerem Glyoxal, monomerem Glyoxal-Monohydrat und monomerem Glyoxal-Dihydrat umfassen.

Vor dem Hintergrund des Vorgenannten kann für eine erfolgreiche Gestaltung der in der EP-A 1 396 484 gegebenen Lehre eine Ausbildung von monomerem Glyoxal-Monohydrat und/oder monomerem Glyoxal-Dihydrat in der Rektifikationskolonne bei der Azeotropdestillation daher nicht ausreichend sein, wenngleich auch diese Hydrate normalerweise eine erhöhte Siedetemperatur aufweisen und sich im Normalfall gemeinsam mit Acrylsäure im Kolonnensumpf anreichern.

Für eine erfolgreiche Gestaltung der in der EP-A 1 396 484 empfohlenen Verfahrensweise bedarf es gemäß eigenen Untersuchungen vielmehr der Ausbildung der Hydrate von "Polyglyoxal" bzw. "Oligoglyoxal". Sie bilden die zweite Gruppe von Glyoxalhydraten. In beispielhafter Weise seien nachfolgend Diglyoxal-Hydrate und Triglyoxal-Hydrate aufgeführt:

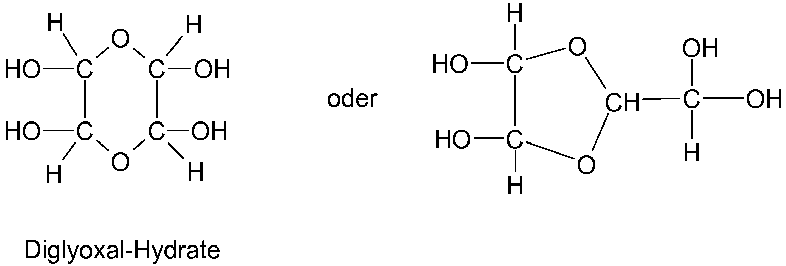

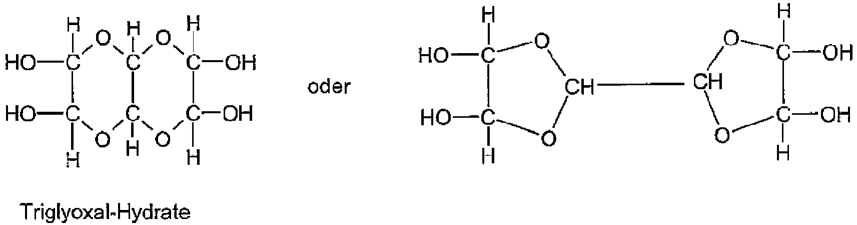

Vermutlich verläuft die Bildung der Polyglyoxal-Hydrate über das monomere Glyoxal-Dihydrat als Zwischenstufe.

Im Unterschied zur Ausbildung der monomeren Glyoxalhydrate bedarf die Ausbildung der Polyglyoxalhydrate erhöhter Temperaturen (in der Regel erfolgt ihre Ausbildung erst bei Temperaturen oberhalb von 50 °C in nennenswertem Umfang) und/oder längerer Reaktionszeiten. Ebenso wie die monomeren Glyoxalhydrate weisen auch die Polyglyoxathydrate die für das monomere Glyoxal typische Polymerisationsförderwirkung bezüglich Acrylsäure nicht mehr oder allenfalls noch in einem wesentlich geringeren Umfang wie selbiges auf. Im Unterschied zur Ausbildung der monomeren Glyoxalhydrate erfolgt die Ausbildung der Polyglyoxalhydrate jedoch weitgehend irreversible (zumindest unter denjenigen Bedingungen, die zur Abtrennung von Acrylsäure aus dem Produktgasgemisch einer heterogen katalysierten partiellen Gasphasenoxidation einer C₃-Vorläuferverbindung der Acrylsäure normalerweise angewendet werden).

Eine erfolgreiche Anwendung der in der EP-A 1 396 484 empfohlenen Verfahrensweise ist somit nur auf der Grundlage der Ausbildung von Polyglyoxalhydraten nachvollziehbar. Selbige bedarf jedoch in nachteiliger Weise sowohl erhöhter Temperaturen als auch erhöhter Verweilzeiten.

EP 1484309 A1 offenbart ein Verfahren zur Herstellung von Acrylsäure aus einer Acrylsäure enthaltenden Lösung mit hoher Konzentration ohne die Durchführung einer azeotrope Destillation. Das Verfahren umfasst die Schritte: Absorbieren eines Acrylsäure enthaltenden Gases, Destillieren der Acrylsäure enthaltenden Lösung ohne die Verwendung eines azeotropen Lösungsmittels, und Zuführen der Rohacrylsäure zu einem Kristallisationsschritt und Schmelzkristallisation der Rohacrylsäure.

Die Aufgabe der vorliegenden Erfindung bestand daher darin, ein gegenüber dem Verfahren des nächstliegenden Standes der Technik verbessertes Verfahren zur Auftrennung von in einem Produktgasgemisch einer partiellen heterogen katalysierten Gasphasenoxidation einer C₃-Vorläuferverbindung der Acrylsäure als Hauptprodukt enthaltener Acrylsäure und als Nebenprodukt enthaltenem Glyoxal zur Verfügung zu stellen, das die beschriebenen Nachteile der Verfahren des Standes der Technik im wesentlichen nicht mehr aufweist und insbesondere keiner Ausbildung von Polyglyoxalhydraten bedarf.

Demgemäß wird ein Verfahren zur Auftrennung von in einem Produktgasgemisch einer partiellen heterogen katalysierten Gasphasenoxidation einer C₃-Vorläuferverbindung der Acrylsäure als Hauptprodukt enthaltener Acrylsäure und als Nebenprodukt enthaltenem Glyoxal, bei dem man eine flüssige Phase P erzeugt, die zu wenigstens 90 % ihres Gewichtes aus Acrylsäure besteht und, bezogen auf die in ihr enthaltene Menge an Acrylsäure, wenigstens 200 mol.ppm Glyoxal (darunter wird in dieser Schrift, wie bereits gesagt, die Gesamtmenge aus monomerem Glyoxal, monomerem Glyoxal-Monohydrat und monomerem Glyoxal-Dihydrat verstanden) enthält, zur Verfügung gestellt, das dadurch gekennzeichnet ist, dass die Trennung des Glyoxals von der Acrylsäure aus der flüssigen Phase P heraus kristallisativ erfolgt, wobei sich die Acrylsäure im gebildeten Kristallisat und das Glyoxal sich in der bei der Kristallisation verbleibenden Mutterlauge anreichert.

Die Vorteilhaftigkeit des erfindungsgemäßen Verfahrens liegt zum einen darin begründet, dass es keine Mengenbeschränkung der Glyoxalnebenproduktbildung fordert bzw. erfordert, und zum anderen darin, dass es nicht der Ausbildung von Polyglyoxalhydraten bedarf.

Ferner überrascht, dass der mit der erfindungsgemäßen kristallisativen Auftrennung einhergehende Abreicherungskoeffizient A^{Gly} des Glyoxal im Regelfall Werte oberhalb von Zehntausend (>> 10 000) erreicht.

Unter dem Abreicherungskoeffizienten A wird generell das Mengenverhältnis von in der Mutterlauge verbliebener Verunreinigung zu im Kristallisat verbliebener Verunreinigung verstanden (jeweils ausgedrückt als Gew.-% bezogen auf die Gesamtmenge an Mutterlauge bzw. die Gesamtmenge an Kristallisat; z. B. durch Zentrifugieren oder durch Zentrifugieren und/oder Waschen können Mutterlauge und Kristallisat im wesentlichen vollständig voneinander getrennt und durch nachfolgende Analyse der Abreicherungskoeffizient A bestimmt werden; eine Mutterlaugenabtrennung zu mehr als 90 Gew.-%, vorzugsweise zu mehr als 95, oder 97 oder 98, oder 99 Gew.-% ihrer Gesamtmenge ist dazu in der Regel ausreichend).

Kein anderes unerwünschtes mögliches Nebenprodukt der heterogen katalysierten partialoxidativen Herstellung von Acrylsäure aus ihren C₃-Vorläuferverbindungen in der Gasphase erreicht im Rahmen einer entsprechenden kristallisativen Auftrennung einen A-Wert vergleichbarer Größe.
Dieser Sachverhalt ist umso bemerkenswerter, als A^{Gly} nicht nur die kristallisative Abtrennung von monomerem Glyoxal, sondern die kristallisative Abtrennung von monomerem Glyoxal, monomerem Glyoxal-Monohydrat und monomerem Glyoxal-Dihydrat beinhaltet (vgl. Definition des alleinstehenden Begriffes "Glyoxal").

Der vorgenannte Befund eröffnet die Möglichkeit, auf dem Weg zu z. B. superabsorbertauglicher Reinacrylsäure die, einer solchen Verwendung im Weg stehenden, Glyoxalverunreinigungen in einem einzigen Trennschritt, in einer einzigen Kristallisationsstufe in befriedigender Weise aus einer flüssigen Phase P abzutrennen.

Die Einheit "mol.ppm" ist so zu verstehen, dass wenn eine bestimmte Menge an flüssiger Phase P z. B. 1 mol an Acrylsäure enthält und dieselbe Menge an flüssiger Phase P gleichzeitig 10 • 10⁻⁶ mol Glyoxal enthält, in dieser Menge an flüssiger Phase P, bezogen auf die in ihr enthaltene molare Menge an Acrylsäure, 10 mol.ppm Glyoxal enthalten sind.

D. h., das erfindungsgemäße Verfahren ist auch dann mit Erfolg anwendbar, wenn die flüssige Phase P, die zu wenigstens 90 % ihres Gewichtes aus Acrylsäure besteht, bezogen auf die in ihr enthaltene molare Menge an Acrylsäure, ≥ 250 mol.ppm, oder ≥ 300 mol.ppm, oder ≥ 400 mol.ppm, oder ≥ 500 mol.ppm, oder ≥ 750 mol.ppm, oder ≥ 1000 mol.ppm, oder ≥ 1250 mol.ppm, oder ≥ 1500 mol.ppm Glyoxal (das ist in dieser Schrift die Gesamtmenge aus monomerem Glyoxal, monomerem Glyoxal-Monohydrat und monomerem Glyoxal-Dihydrat) enthält.

Im Regelfall wird die flüssige Phase P, die zu wenigstens 90 % ihres Gewichtes aus Acrylsäure besteht, bezogen auf die in ihr enthaltene molare Menge an Acrylsäure, ≤ 5 mol.-%, häufig ≤ 2 mol.-%. oder ≤ 1 mol.% Glyoxal enthalten. Selbstredend ist das erfindungsgemäße Verfahren aber auch noch im Fall der vorgenannten Glyoxalgehalte erfolgreich anwendbar.

Das erfindungsgemäße Verfahren ist aber für alle (d. h. jeden einzelnen, auch die vorgenannten) in dieser Schrift indiviualisiert genannten Glyoxalgehalte der flüssigen Phase P (jeweils angegeben in mol.ppm bezogen auf die in der flüssigen Phase P enthaltene molare Menge an Acrylsäure) erfolgreich anwendbar, wenn die flüssige Phase P zu ≥ 90 Gew.-%, oder zu ≥ 95 Gew.-%, oder zu ≥ 96 Gew.-%, oder zu ≥ 97 Gew.-%, oder zu ≥ 98 Gew.-%, oder zu ≥ 99 Gew.-% (jeweils des Gewichts der flüssigen Phase P) aus Acrylsäure besteht.

Der Gehalt einer erfindungsgemäß zu behandelnden flüssigen Phase P (oder einer sonstigen flüssigen Phase) an Glyoxal (d. h., der Gesamtgehalt der flüssigen Phase P an monomerem Glyoxal, monomerem Glyoxal-Monohydrat und monomerem Glyoxal-Dihydrat) wird im Sinne dieser Schrift wie folgt bestimmt:
Zunächst wird eine Derivatisierungslösung D hergestellt. Dazu werden 2,0 g einer 50 gew.-%igen Lösung von 2,4-Dinitrophenylhydrazin (Hersteller: Aldrich, Reinheit 97%) bei einer Temperatur von 25 °C in 62 ml einer 37,0 gew.-%igen wässrigen Salzsäure gelöst (Hersteller: Aldrich, Reinheit: ≥ 99,999%). Die dabei resultierende Lösung wird anschließend (ebenfalls bei einer Temperatur von 25 °C) in 335 g destilliertes Wasser eingerührt. Nach 1-stündigem Rühren bei 25 °C wird durch Abfiltrieren die Derivatisierungslösung D als das anfallende Filtrat erhalten.

Zur Bestimmung des Gehaltes einer flüssigen Phase P an Glyoxal wird 1 g (bei Bedarf kann diese Menge in entsprechender Weise erhöht werden) der Derivatisierungslösung D in ein Gewindeschraubglas eingewogen, dessen Fassungsvermögen 10 ml beträgt. Anschließend wird in das so befüllte Gewindeschraubglas eine Probe der flüssigen Phase P zugewogen, deren Menge im Bereich 0,15 bis 2,0 g liegt.

Durch Schütteln wird der Gesamtinhalt des Gewindeschraubglases anschließend gemischt und nachfolgend während eines Zeitraums von 10 Minuten bei einer Temperatur von 25 °C sich selbst überlassen. Während dieser Zeit bildet sich aus dem im Gewindeschraubglas enthaltenen monomeren Glyoxal durch chemische Reaktion mit 2,4-Dinitrophenylhydrazin das entsprechende Hydrazon H von monomerem Glyoxal. Während dieser Zeit entzieht das 2,4-Dinitrophenylhydrazin aber auch aus den im Gewindeschraubglas enthaltenen monomeren Glyoxal-Monohydrat und Glyoxal-Dihydrat das in selbigen gebundene monomere Glyoxal in Form des Hydrazons H (ein entsprechender Entzug von monomerem Glyoxal aus im Gewindeschraubglas enthaltenen Polyglyoxal-Hydraten findet dagegen im wesentlichen nicht statt).

Durch Zugabe von 0,5 g Eisessig (Hersteller: Aldrich, Reinheit: ≥ 99,8%) ins Gewindeschraubglas wird anschließend die erfolgte Hydrazonbildung eingefroren. Geht mit der Essigsäurezugabe eine Ausbildung von festem Niederschlag einher, wird sukzessive weitere Essigsäure zugegeben, um die Niederschlagbildung wieder aufzulösen (die insgesamt zugegebene Essigsäuremenge darf jedoch 1,0 g nicht überschreiten). Ist der gebildete Niederschlag auch bei Erreichen der Höchstgrenze (1,0 g) der erlaubten Essigsäuregesamtmengenzugabe nicht in Lösung gegangen, werden 0,5 g Dimethylphthalat zugewogen. Vermögen auch diese den gebildeten Niederschlag nicht aufzulösen, wird die Dimethylphthalatzugabemenge sukzessive erhöht, um diese Auflösung zu bewirken (die insgesamt zugegebene Dimethylphthalatmenge darf jedoch 1,0 g nicht überschreiten). Ist der gebildete Niederschlag auch bei Erreichen der Höchstgrenze (1,0 g) der erlaubten Dimethylphthalatgesamtmengenzugabe nicht in Lösung gegangen, werden 2 g eines Gemisches G aus 9 g Acetonitril und 1 g Dimethylphthalat zugegeben. Vermag auch diese Zugabe den Niederschlag nicht aufzulösen, wird die Zugabemenge an Gemisch G sukzessive erhöht, um diese Auflösung zu bewirken. Normalerweise überschreitet die insgesamt zugegebene Menge an Gemisch G 5 g nicht, um die Auflösung des Niederschlags zu bewirken (alle vorgenannten Auflösungsversuche werden bei 25 °C durchgeführt).

Die wie beschrieben im Gewindeschraubglas erzeugte Lösung des Hydrazons H wird anschließend unter Anwendung der nachfolgenden Betriebsbedingungen mittels HPLC (High Pressure Liquid Chromatograpy) auf ihren Hydrazongehalt untersucht (aus der molaren Menge desselben resultiert unmittelbar die molare Menge an in der flüssigen Phase P enthaltenem Glyoxal):

| | |
|---|---|
| zu verwendende Chromatographiesäule | Waters Symmetry C18, 150 x 4,6 mm, 5 µm |
| | (der Fa. Waters Associates, Milford, Massachusetts, USA); |
| Injektionsvolumen der zu analysierenden Lösung: 50 µl (Zeitpunkt t = 0); | |
| Temperatur: 40 °C; | |
| Eluentstrom: 1,5 ml/min; | |
| Analysendauer: 17 min; | |
| Equilibrierdauer: 8 min; | |
| Eluent: | im Zeitraum t > 0 min bis 15 min ein Gemisch aus 30 Gew.-% Acetonitril, 50 Gew.-% Wasser und 20 Gew.-% Tetrahydrofuran (jeweils HPLC-grade); |
| | im Zeitraum > 15 min bis 17 min ein Gemisch aus 65 Gew.-% Acetonitril, 30 Gew.-% Wasser und 5 Gew.-% Tetrahydrofuran; |
| | im Zeitraum > 17 min bis 25 min ein Gemisch aus 30 Gew.-% Acetonitril, 50 Gew.-% Wasser und 20 Gew.-% Tetrahydrofuran (anschließend ist die Kolonne equilibriert und wieder startbereit für die nächste Analyse). |

Die Retentionszeit des Glyoxals als Hydrazon H beträgt unter vorgenannten Bedingungen 7,613 min.

Die Analyse erfolgt mittels monochromatischer Strahlung der Wellenlänge 365 nm. Als Analysenmethode wird die Absorptionsspektroskopie angewendet.
Die Variation des Eluents über die Eluationsdauer gewährleistet eine erhöhte Trennwirkung (in der Regel enthält die flüssige Phase P neben Glyoxal noch andere Nebenproduktaldehyde und/oder Nebenproduktketone, die mit 2,4-Dinitrophenylhydrazin das jeweilige entsprechende Hydrazon bilden).

Zur Eichung des HPLC-Verfahrens wird man anwendungstechnisch zweckmäßig eine Lösung von monomerem Glyoxal in Methanol einsetzen, die 50 Gew.ppm monomeres Glyoxal enthält.

Sie wird zu diesem Zweck wie vorstehend beschrieben mittels der Derivatisierungslösung D behandelt und anschließend der beschriebenen HPLC-Analyse unterworfen.

Das erfindungsgemäße Verfahren besticht unter anderem, wie bereits gesagt, dadurch, dass es nicht auf den Einsatz von hochreinen C₃-Vorläuferverbindungen der Acrylsäure für die heterogen katalysierte partielle Gasphasenoxidation zur Herstellung derselben angewiesen ist.

Beispielsweise kann für die heterogen katalysierte partielle Gasphasenoxidation zur Herstellung der Acrylsäure ein Reaktionsgasausgangsgemisch eingesetzt werden, das, bezogen auf die in ihm enthaltene molare Menge an der eingesetzten C₃-Vorläuferverbindung (z. B. Propan, Propylen, Acrolein, Propionsäure, Propionaldehyd, Propanol und/oder Glyzerin), eine molare Gesamtmenge an C₂-Verbindungen (z. B. Ethan, Ethylen, Acetylen, Acetaldehyd, Essigsäure und/oder Äthanol) von ≥ 200 mol.ppm, oder ≥ 250 mol.ppm, oder ≥ 300 mol.ppm, oder ≥ 400 mol.ppm, oder ≥ 500 mol.ppm, oder ≥ 750 mol.ppm, oder ≥ 1000 mol.ppm, oder ≥ 1250 mol.ppm, oder ≥ 1500 mol.ppm aufweist.

Das Reaktionsgasausgangsgemisch ist dabei dasjenige Gasgemisch, das dem Katalysatorbett zum Zweck der Partialoxidation der in ihm enthaltenen C₃-Vorläuferverbindung zu Acrylsäure zugeführt wird. Neben der C₃-Vorläuferverbindung, unerwünschten Verunreinigungen sowie molekularem Sauerstoff als Oxidationsmittel enthält das Reaktionsgasausgangsgemisch in der Regel noch inerte Verdünnungsgase wie z. B. N₂, CO₂, H₂O, Edelgas, molekularen Wasserstoff etc.. Jedes inerte Verdünnungsgas ist normalerweise so beschaffen, dass es zu wenigstens 95 mol-% seiner Ausgangsmenge im Verlauf der heterogen katalysierten Partialoxidation unverändert erhalten bleibt.

Der Anteil der C₃-Vorläuferverbindung am Reaktionsgasausgangsgemisch kann z. B. im Bereich von 4 bis 20 Vol.-%, oder von 5 bis 15 Vol.-%, oder von 6 bis 12 Vol.-% liegen.

Normalerweise enthält das Reaktionsgasausgangsgemisch, bezogen auf die Stöchiometrie der Partialoxidationsreaktion der C₃-Vorläuferverbindung zu Acrylsäure, einen Überschuss an molekularem Sauerstoff, um die in der Regel oxidischen Katalysatoren wieder zu reoxidieren.

Dieser Überschuss kann im Fall einer nachfolgenden Anwendung der erfindungsgemäßen Verfahrensweise besonders hoch gewählt werden, da mit zunehmendem Sauerstoffüberschuss in der Regel auch eine Zunahme der unerwünschten Nebenkomponentenbildung an Glyoxal einhergeht.

In gleicher Weise kann bei der heterogen katalysierten partiellen Gasphasenoxidation der C₃-Vorläuferverbindung zur Acrylsäure die im Katalysatorbett vorliegende maximale Reaktionstemperatur vergleichsweise erhöht gewählt werden, wenn im Anschluss an die Partialoxidation das erfindungsgemäße Verfahren zur Anwendung kommt. Dies ist u. a. darauf zurückzuführen, dass mit zunehmender Maximaltemperatur in der Regel auch eine Zunahme der unerwünschten Nebenkomponentenbildung an Glyoxal einhergeht. Die Anwendung erhöhter Maximaltemperaturen gestattet in der Regel jedoch den Einsatz von Katalysatoren mit geringerer Aktivität, was die Möglichkeit einer verlängerten Katalysatorstandzeit eröffnet. Allerdings erfolgt bei Verwendung von Katalysatoren mit geringerer Aktivität mit zunehmendem Umsatz der C₃-Vorläuferverbindung in zunehmendem Umfang häufig auch unerwünschte Vollverbrennung derselben. Als Zwischenprodukt kann dabei gegebenenfalls ebenfalls Glyoxal gebildet werden.

In ähnlicher Weise kann im Kontext mit der erfindungsgemäßen Verfahrensweise auch bei der Auswahl der Belastung des Katalysatorbetts mit C₃-Vorläuferverbindung großzügiger verfahren werden.
Des Weiteren hat sich gezeigt, dass die Glyoxalnebenproduktbildung durch erhöhte Wasserdampfgehalte im Reaktionsgasgemisch begünstigt wird. Das erfindungsgemäβe Verfahren ist deshalb nicht zuletzt dann von Relevanz, wenn das für die heterogen katalysierte partielle Gasphasenoxidation der C₃-Vorläuferverbindung eingesetzte Reaktionsgasausgangsgemisch ≥ 1 Gew.-%, oder ≥ 2 Gew.-%, oder ≥ 3 Gew.-%, oder ≥ 4 Gew.-%, oder ≥ 5 Gew.-%, oder ≥ 7 Gew.-%, oder ≥ 9 Gew.-%, oder ≥ 15 Gew.-%, oder ≥ 20 Gew.-% an Wasserdampf enthält. Im Regelfall wird der Wasserdampfgehalt des Reaktionsgasausgangsgemischs jedoch nicht mehr als 40 Gew.-%, häufig nicht mehr als 30 Gew.-% betragen.

Im Übrigen kann das Verfahren der heterogen katalysierten partiellen Gasphasenoxidation zur Herstellung der Acrylsäure in an sich bekannter Weise wie im Stand der Technik beschrieben durchgeführt werden.

Handelt es sich bei der C₃-Vorläuferverbindung z. B. um Propylen und/oder Acrolein, kann die heterogen katalysierte partielle Gasphasenoxidation z. B. wie in den Schriften WO 2005/042459, WO 2005/047224 und WO 2005/047226 beschrieben durchgeführt werden.

Ist die C₃-Vorläuferverbindung z. B. Propan, kann die heterogen katalysierte partielle Gasphasenoxidation zur Herstellung der Acrylsäure z. B. wie in den Schriften
EP-A 608 838, DE-A 198 35 247, DE-A 102 45 585 und DE-A 102 46 119 beschrieben durchgeführt werden.

Ist die C₃-Vorläuferverbindung z. B. Glycerin, kann die heterogen katalysierte partielle Gasphasenoxidation zur Herstellung der Acrylsäure z. B. wie in den Schriften WO 2007/090991, WO 2006/114506, WO 2005/073160, WO 2006/114506, WO 2006/092272 oder WO 2005/073160 beschrieben durchgeführt werden.

Es wurde auch schon vorgeschlagen, das Propylen als C₃-Vorläuferverbindung durch eine der partiellen Gasphasenoxidation vorgeschaltete partielle Dehydrierung und/oder Oxidehydrierung von Propan zu erzeugen (z. B. WO 076370, WO 01/96271, EP-A 117146, WO 03/011804 und WO 01/96270).

Zur Abtrennung der Acrylsäure aus dem Produktgasgemisch der heterogen katalysierten partiellen Gasphasenoxidation einer C₃-Vorläuferverbindung wird normalerweise grundsätzlich eine Kombination unterschiedlicher Trennverfahren angewendet, um auf möglichst wirtschaftliche Weise eine dem nachfolgenden Verwendungszweck der Acrylsäure angemessene Reinheit derselben zu erzielen. Die im Einzelnen angewandte Kombination ist dabei nicht zuletzt von der Art und Menge der im Produktgasgemisch enthaltenen, von Acrylsäure verschiedenen Bestandteile abhängig. Die Erzeugung einer erfindungsgemäß behandelbaren flüssigen Phase P kann daher auf unterschiedlichsten Wegen erfolgen.

Wesentlicher Bestandteil einer solchen Kombination von Trennverfahren sind normalerweise nicht kristallisative thermische Trennverfahren. Bei den nicht kristallisativen thermischen Trennverfahren handelt es sich um solche Trennverfahren, bei denen in trennwirksame Einbauten enthaltenden Trennkolonnen gasförmige (aufsteigend) und flüssige (absteigend) Stoffströme bzw. zwei flüssige Stoffströme im Gegenstrom geführt werden, wobei infolge der zwischen den Stoffströmen bestehenden Gradienten ein Wärme- und Stoffaustausch stattfindet, der letztlich die in der Trennkolonne gewünschte Trennung bedingt.

Beispiele für solche nicht kristallisative thermische Trennverfahren sind die (partielle) Kondensation, die fraktionierende Kondensation (vgl. DE-A 199 24 532) und die Rektifikation.

Die resultierende Trennwirkung beruht hier vor allem auf der Verschiedenheit der Siedepunkte von Acrylsäure und den von Acrylsäure verschiedenen Nebenkomponenten. Ein weiteres Beispiel bildet die Absorption. Die Trennwirkung beruht hier insbesondere auf der unterschiedlichen Löslichkeit von Acrylsäure und den von Acrylsäure verschiedenen Nebenkomponenten in der Absorptionsflüssigkeit. Das Vorgenannte trifft auch auf die nicht kristallisativen thermischen Trennverfahren der Strippung (ein Strippgas nimmt aus einer Flüssigkeit in selbiger gelöst enthaltene Bestandteile mit unterschiedlicher Affinität auf) und der Desorption (der Umkehrprozess zur Absorption; in der Flüssigphase Gelöstes wird durch Partialdruckerniedrigung abgetrennt) zu. Der Begriff der thermischen Trennverfahren umfasst aber auch die azeotrope Destillation bzw. Rektifikation (sie nützen die unterschiedlich ausgeprägte Neigung von Acrylsäure und den Nebenkomponenten (den von Acrylsäure verschiedenen Bestandteilen im Reaktionsgasgemisch der Partialoxidation) zur Ausbildung von Azeotropen mit zugesetzten azeotropen Schleppern aus). Weiterhin umfasst der Begriff der nicht kristallisativen thermischen Trennverfahren die Extraktion.

Ein im wesentlichen allen möglichen Kombinationen thermischer Trennverfahren zur Abtrennung von Acrylsäure aus dem Produktgasgemisch der heterogen katalysierten partiellen Gasphasenoxidation einer C₃-Vorläuferverbindung gemeinsames Merkmal ist, dass, gegebenenfalls nach direktem und/oder indirektem Abkühlen des vorgenannten Produktgasgemischs, im Produktgasgemisch enthaltene Acrylsäure in einem Grundabtrennschritt in die kondensierte (insbesondere flüssige) Phase überführt wird (von dem im Rahmen einer solchen Grundabtrennung gasförmig verbleibenden Restgas wird anwendungstechnisch zweckmäßig wenigstens eine Teilmenge in die partielle heterogen katalysierte Gasphasenoxidation der C₃-Vorläuferverbindung (z.B. in das Reaktionsgasausgangsgemisch derselben) als "Kreisgas" zurückgeführt ("Kreisgasfahrweise"); im Regelfall besteht Restgas (Kreisgas) überwiegend aus den für die partielle heterogen katalysierte Gasphasenoxidation der C₃-Vorläuferverbindung mitverwendeten inerten Verdünnungsgasen sowie aus bei der Partialoxidation üblicherweise als Nebenprodukt gebildetem Wasserdampf und durch auf dem Weg der unerwünschten vollständigen Oxidation der C₃-Vorläuferverbindung entstehenden Nebenprodukten (z.B. Kohlenoxiden), die auch Glyoxal umfassen können; ist die Grundabtrennung eine Absorption, kann das Kreisgas auch Absorptionsmittel umfassen; teilweise enthält es noch geringe Mengen an bei der Partialoxidation nicht verbrauchtem molekularem Sauerstoff (Restsauerstoff) und/oder an nicht umgesetzter organischer C₃-Vorläuferverbindung (vgl. z.B. WO 2004/007405 und DE-A 102007019597)).

Dies kann z. B. durch Absorption in ein geeignetes Lösungsmittel (z. B. Wasser, hochsiedende organische Lösungsmittel, wässrige Lösungen) und/oder durch partielle oder im wesentlichen vollständige Kondensation (z. B. fraktionierende Kondensation) erfolgen (vgl. dazu z. B. die Schriften EP-A 13 88 533, EP-A 13 88 532, DE-A 102 35 847, EP-A 79 28 67, WO 98/01415, EP-A 10 15 411, EP-A 10 15 410, WO 99/50219, WO 00/53560, WO 02/09839, DE-A 102 35 847, WO 03/041833, DE-A 102 23 058, DE-A 102 43 625, DE-A 103 36 386, EP-A 85 41 29, US-A 4,317,926, DE-A 198 37 520, DE-A 196 06 877, DE-A 190 50 1325, DE-A 102 47 240, DE-A 197 40 253, EP-A 69 57 36, EP-A 98 22 87, EP-A 10 41 062, EP-A 11 71 46, DE-A 43 08 087, DE-A 43 35 172, DE-A 44 36 243, DE-A 19 924 532, DE-A 103 32 758 sowie DE-A 19 924 533). Eine Acrylsäureabtrennung kann auch wie in der EP-A 98 22 87, der EP-A 98 22 89, der DE-A 103 36 386, der DE-A 101 15 277, der DE-A 196 06 877, der DE-A 197 40 252, der DE-A 196 27 847, der EP-A 92 04 08, der EP-A 10 68 174, der EP-A 10 66 239, der EP-A 10 66 240, der WO 00/53560, der WO 00/53561, der DE-A 100 53 086 und der EP-A 98 22 88 vorgenommen werden. Günstige Abtrennweisen sind auch die in den Schriften WO 2004/063138, WO 2004/035514, DE-A 102 43 625 und DE-A 102 35 847 beschriebenen Verfahren.

Die weiteren anzuwendenden Trennschritte, um aus der im Rahmen der beschriebenen Grundabtrennung erhaltenen, das Zielprodukt Acrylsäure enthaltenden flüssigen (bzw. allgemein kondensierten) Phase die Acrylsäure im gewünschten Reinheitsgrad abzutrennen, können je nach Zielvorgabe unterschiedlichste Kombinationen von adsorptiven, extraktiven, desorptiven, destillativen, strippenden, rektifikativen, azeotropdestillativen, azeotrop rektifikativen sowie kristallisativen Verfahren sein.

Wird dabei eine flüssige Phase P durchlaufen, oder ist bereits die im Rahmen der beschriebenen Grundabtrennung anfallende, das Zielprodukt Acrylsäure enthaltende, flüssige Phase eine flüssige Phase P, kann das erfindungsgemäße Verfahren zur Abtrennung von in der flüssigen Phase P enthaltenem Glyoxal in vorteilhafter Weise zur Anwendung kommen. Die Tatsache, dass bei Anwendung der erfindungsgemäßen Verfahrensweise auf eine flüssige Phase P (d. h., beim Abkühlen einer flüssigen Phase P) regelmäßig Acrylsäure auskristallisiert, ist durch deren hohen geforderten Mindestgehalt an Acrylsäure bedingt. Dieser kann, wie bereits gesagt, z. B. ≥ 90 bis ≥ 98 Gew.-%, oder ≥ 93 bis ≥ 97 Gew.-% betragen.

Dabei kann das erfindungsgemäße Kristallisationsverfahren auf gleiche Weise ausgeübt und auf gleiche Weise in das Gesamtverfahren zur Abtrennung von (Rein) Acrylsäure aus dem Produktgasgemisch der heterogen katalysierten partiellen Gasphasenoxidation der C₃-Vorläuferverbindung integriert sein, wie es insbesondere die folgenden Schriften des Standes der Technik lehren: die WO 02/055469, die WO 03/078378, die WO 01/77056, die WO 03/041833, die DE-A 196 06 877, die DE-A 103 36 386, die WO 98/01414, die WO 01/77056, die EP-A 1 484 308, die EP-A 1 484 309, die US-A 2004/0242826, die DE-A 102 43 625, die DE-A 196 06 877, die EP-A 792 867, die EP-A 1 015 410, die EP-A 920 408, die EP-A 1 189 861, die EP-A 1 015 411, die EP-A 1 068 174, die WO 2004/035514, die EP-A 1 066 293, die EP-A 1 163 201, die EP-A 1 159 249, die WO 02/090310, die DE-A 101 22 787, die WO 03/041832, die DE-A 102 35 847, die EP-A 1 252 129, die EP-A 616 998, die EP-A 1 388 533, die EP-A 1 125 912 und die EP-A 1 116 709.

Von ganz besonderer Bedeutung ist das erfindungsgemäße Verfahren dann, wenn man die erfindungsgemäß zu behandelnde, Acrylsäure als Hauptbestandteil sowie Glyoxal als Nebenprodukt enthaltende flüssige Phase P aus dem Produktgasgemisch einer heterogen katalysierten partiellen Gasphasenoxidation wenigstens eines C₃-Vorläufers der Acrylsäure unter Anwendung wenigstens eines nicht kristallisativen thermischen Trennverfahren erzeugt Dies insbesondere dann, wenn man bei der erfindungsgemäßen kristallisativen Abtrennung des in der flüssigen Phase P enthaltenen Glyoxals dabei verbleibende Mutterlauge (die das Glyoxal dann angereichert enthält) in wenigstens eines der zur Herstellung der flüssigen Phase P angewandten nicht kristallisativen thermischen Trennverfahren rückführt.

Die Grundstruktur einer solchen kombinierten Anwendung von nicht kristallisativen thermischen Trennverfahren und kristallisativern Trennverfahren lehren z. B. die DE-A 196 06 877, die EP-A 792 867 sowie die EP-A 1 484 308, die EP-A 1 484 309, die EP-A 1 116 709 und insbesondere die EP-A 1 015 410.

Das erfindungsgemäße Verfahren ist im Fall einer solchen Kombination insofern von erhöhter Bedeutung, als sich beim kontinuierlichen Betrieb einer solchen Verfahrensweise das Glyoxal in der erfindungsgemäß zu behandelnden flüssigen Phase P durch die Mutterlaugenrückführung aufpegelt, da die Mutterlauge (wie bereits erwähnt) das Glyoxal angereichert enthält. D. h., auch vergleichsweise geringe Glyoxalgehalte im Produktgasgemisch der Gasphasenoxidation können sich so zu einem ernsten Problem auswachsen (aus einer zunächst nicht flüssigen Phase P (die zunächst weniger als 200 mol.ppm Glyoxal bezogen auf die darin enthaltene molare Menge an Acrylsäure enthält), kann im Verlauf der kontinuierlichen Betriebsdauer eine erfindungsgemäß zu behandelnde flüssige Phase P (die dann wenigstens 200 mol.ppm Glyoxal bezogen auf die darin enthaltene molare Menge an Acrylsäure enthält) unter Umständen erst erwachsen). Ein überdurchschnittlicher Abreicherungskoeffizient A^{Gly} ist in diesen Fällen unabdingbar für eine erfolgreiche Verfahrensdurchführung.

Der erfindungsgemäß erforderliche erhöhte Glyoxalgehalt in flüssigen Phasen P kann in selbigen aber auch z. B. dann enthalten sein, wenn bei der Kristallisation von flüssigen, Acrylsäure enthaltenden, Phasen, die lediglich geringere Glyoxalgehalte aufweisen, anfallende Mutterlaugen zum Zweck der Ausbeutesteigerung weiterkristallisiert werden, oder wenn in nicht kristallisativen thermischen Trennverfahren auf dem Weg zur Herstellung von reiner Acrylsäure anfallende, gegebenenfalls in erfindungsgemäβer Weise verunreinigte, Nebenstoffströme zum Zweck der Ausbeutesteigerung erfindungsgemäß behandelt werden.

Da bei der heterogen katalysierten partiellen Gasphasenoxidation von C₃-Vorläuferbindungen Wasser normalerweise zwingend als Nebenprodukt gebildet sowie gegebenenfalls zusätzlich als inertes Verdünnungsgas im Reaktionsgasgemisch mitverwendet wird, enthält die erfindungsgemäß zu behandelnden flüssige Phase P häufig nicht nur Wasser, sondern neben monomerem Glyoxal gleichzeitig auch sowohl monomeres Glyoxal-Monohydrat und monomeres Glyoxal-Dihydrat. In manchen Fällen kann in einer erfindungsgemäß zu behandelnden, flüssigen Phase P aber auch nur monomeres Glyoxal enthalten sein (der Vorzug der erfindungsgemäßen Verfahrensweise besteht unter anderem darin, dass sie in beiden Fällen wirksam ist).

D.h., das erfindungsgemäße Verfahren ist insbesondere auch dann anwendbar, wenn das in der flüssigen Phase P enthaltene Glyoxal zu wenigstens 30 mol-%, oder zu wenigstens 50 mol-%, oder zu wenigstens 70 mol-%, oder zu wenigstens 90 mol-%, oder zu wenigstens 95 mol-% als monomeres Glyoxal-Monohydrat und/oder monomeres Glyoxal-Dihydrat in der flüssigen Phase P vorliegt (bzw. in der flüssigen Phase P enthalten ist).

Häufig enthält die flüssige Phase P, bezogen auf die darin enthaltene Acrylsäuremenge, 0,20 bis 30, oder 0,20 bis 20, oder 0,20 bis 10 Gew.-% an Wasser (das Hydratwasser (von z. B. Glyoxalhydraten) ist in diese Wassermenge eingerechnet). Vielfach beträgt der vorgenannte Wassergehalt der flüssigen Phase P, bezogen auf die darin enthaltene Acrylsäuremenge, 0,50 bis 30 Gew.-%, oder 0,50 bis 20 Gew.-% oder 0,50 bis 10 Gew.-%.

Bei dem wenigstens einen zur Erzeugung der erfindungsgemäß zu behandelnden flüssigen Phase P aus dem Produktgasgemisch einer heterogen katalysierten partiellen Gasphasenoxidation eines C₃-Vorläufers der Acrylsäure angewandten nicht kristallisativen thermischen Trennverfahren (in das bei der nachfolgenden erfindungsgemäßen kristallisativen Abtrennung des in der flüssigen Phase P enthaltenen Glyoxals verbleibende Mutterlauge (die das Glyoxal dann angereichert enthält) wenigstens teilweise rückgeführt werden kann) wird es sich im Regelfall um eine Rektifikation, azeotrope Rektifikation, Absorption, Adsorption, Extraktion, Desorption, Destraktion, partielle Kondensation, Strippung, fraktionierende Kondensation oder um eine Kombination aus mehreren dieser Verfahren handeln. Häufig wird man zur Erzeugung der erfindungsgemäß zu behandelnden flüssigen Phase P vorgenannte Verfahren mehrfach anwenden.

Im einfachsten Fall kann die erfindungsgemäß zu behandelnde flüssige Phase P das Absorbat und/oder partielle und/oder fraktionierend erhaltene Kondensat einer absorptiven und/oder kondensativen Abtrennung der Acrylsäure aus dem Produktgasgemisch einer heterogen katalysierten partiellen Gasphasenoxidation wenigstens eines der in dieser Schrift aufgeführten C₃-Vorläufer sein. Erfindungsgemäß bevorzugt erfolgt dann die Rückführung der Glyoxal angereichert enthaltenden Mutterlauge in die Absorption und/oder (gegebenenfalls fraktionierte) Kondensation. In zweckmäßiger Weise weist eine wie beschrieben anzuwendende vernetzte Betriebsweise aus wenigstens einem nicht kristallisativen thermischen Trennverfahren zur Erzeugung der erfindungsgemäß zu behandelnden flüssigen Phase P und der erfindungsgemäßen kristallisativen Glyoxalabtrennung aus der so erzeugten flüssigen Phase P, bei der bei der kristallisativen Glyoxalabtrennung anfallende, Glyoxal angereichert enthaltende Mutterlauge wenigstens teilweise in wenigstens eines der zur Erzeugung der erfindungsgemäß zu behandelnden flüssigen Phase P angewandten nicht kristallisativen thermischen Trennverfahren rückgeführt wird, einen Auslass für wenigstens einen Glyoxal angereichert enthaltenden Stoffstrom auf.

In vorteilhafter Weise liegt dieser auf der Seite der nicht kristallisativen thermischen Trennverfahren. In der Regel wird man als solchen Auslass die Sumpfflüssigkeit einer Trennkolonne verwenden, aus der die erfindungsgemäß zu behandelnde flüssige Phase P selbst oder der im weiteren Verlauf in die erfindungsgemäß zu behandelnde flüssige Phase P zu wandelnde Stoffstrom z. B. über Seitenentnahme entnommen wird (generell sollte ein solcher Auslass unterhalb der vorgenannten Seitenentnahme liegen). Ist die erfindungsgemäß zu behandelnde flüssige Phase P z. B. eine wie in den Schriften PCT/EP2008/050785, DE-A 102007055086 bzw. EP-A 1 554 234 beschrieben über Seitenentnahme aus dem Produktgasgemisch der heterogen katalysierten partiellen Gasphasenoxidation der C₃-Vorläuferverbindung gewonnene Kondensatfraktion, kann als der oben angesprochene Glyoxalauslass auch der Auslass aus der Rückspaltvorrichtung (dem Spaltgefäß) für die Michael-Addukt-Acrylsäureoligomeren fungieren. Dabei macht sich vorteilhaft bemerkbar, dass Glyoxal bei erhöhter Temperatur (in Abwesenheit von besonderen Rückspaltkatalysatoren) primär in die erwähnten Polyglyoxale bzw. deren Hydrate gewandelt wird.

In der Regel gilt, dass wenn die erfindungsgemäß zu behandelnde flüssige Phase P aus einer Trennkolonne z. B. über Seitenentnahme entnommen wird, in welcher ein nicht kristallisatives thermisches Trennverfahren im Beisein eines in der Trennkolonne von oben nach unten geführten wässrigen Absorptionsmittels und/oder einer wässrigen Rücklaufflüssigkeit durchgeführt wird, der Glyoxalgehalt in der entnommenen flüssigen Phase P um so größer ist, je weiter unten die Entnahmestelle sich in der Trennkolonne befindet.

Ein Glyoxalauslass kann sich aber auch oder nur auf der Seite der erfindungsgemäßen Abtrennung, d. h., auf der kristallisativen Seite befinden. In diesem Fall wird der Auslass normalerweise aus Glyoxal angereichert enthaltender Mutterlauge bestehen.

Wird die erfindungsgemäße Abtrennung z. B. mittels einer Kombination von dynamischer und statischer Kristallisation gemäß der EP-A 616 998 ausgeführt, wird sich der Glyoxal angereichert enthaltende Glyoxalauslass in der Regel (anwendungstechnisch zweckmäßig) im Bereich der statischen Kristallisation befinden.

Letzteres insbesondere dann, wenn bei Anwendung des erfindungsgemäßen Verfahrens keine Rückführung von Glyoxal angereichert enthaltender Mutterlauge in wenigstens ein nicht kristallisatives thermisches Trennverfahren durchgeführt wird.

Das erfindungsgemäße Verfahren ist nicht zuletzt dann günstig, wenn die erfindungsgemäß zu behandelnde flüssige Phase P (z. B. über eine der vorstehend beschriebenen Verfahrensweisen) auf ein Produktgasgemisch einer heterogen katalysierten partiellen Gasphasenoxidation eines C₃-Vorläufers der Acrylsäure zurückgeht, das, bezogen auf die im Produktgasgemisch enthaltene molare Menge an Acrylsäure, wenigstens 200 mol.ppm Glyoxal, oder ≥ 250 mol.ppm Glyoxal, oder ≥ 300 mol.ppm Glyoxal, oder ≥ 400 mol.ppm Glyoxal, oder ≥ 500 mol.ppm Glyoxal, oder ≥ 750 mol.ppm Glyoxal, oder ≥ 1000 mol.ppm Glyoxal, oder ≥ 1250 mol.ppm Glyoxal, oder ≥ 1500 mol.ppm Glyoxal enthält.

Im Normalfall werden die vorgenannten Glyoxalgehalte des Produktgasgemischs (in gleicher Weise bezogen) ≤ 5 mol.-% betragen. Vielfach wird der Acrylsäuregehalt der vorgenannten Produktgasgemische 1 bis 30 Vol.-% betragen.

Das erfindungsgemäße Verfahren ist insbesondere auch dann anwendbar, wenn man die erfindungsgemäß zu behandelnde flüssige Phase P dadurch gewinnt, dass man das, zuvor durch direkten und/oder indirekten Wärmeaustausch gegebenenfalls abgekühlte, Produktgasgemisch einer Absorption der Acrylsäure aus dem Produktgasgemisch heraus mit einer wässrigen Lösung oder mit Wasser unterwirft (vgl. z. B. EP-A 1 388 532 sowie EP-A 1 388 533). Das dabei erhaltene, die Acrylsäure enthaltende wässrige Absorbat kann unmittelbar die erfindungsgemäß zu behandelnde flüssige Phase P sein.

Sollte der Acrylsäuregehalt des wässrigen Absorbats jedoch noch unterhalb von 90 Gew.-% (bezogen auf das Gewicht des Absorbats) liegen - oder aber auch aus anderen Gründen - kann man das wässrige Absorbat (gegebenenfalls nach einer zuvor erfolgten Desorption und/oder Strippung von einen geringeren Siedepunkt als Acrylsäure aufweisenden Bestandteilen im Absorbat) einer azeotropen Destillation (Rektifikation) zur Entfernung wenigstens einer Teilmenge des im Absorbat enthaltenen Wassers unterwerfen und anschließend von der dabei verbleibenden Restmenge (als flüssige Phase P) der erfindungsgemäßen kristallisativen Abtrennung unterwerfen. Als diesbezüglich geeignete Schleppmittel seien beispielsweise Heptan, Dimethylcyclohexan, Ethylcyclohexan, Toluol, Ethylbenzol, Octan, Chlorbenzol, Xylol oder Mischungen (z. B. aus 60 Gew.-% Toluol und 40 Gew.-% Heptan) genannt.

Als alternative Schleppmittel können aber auch Methylisobutylketon oder Isopropylacetat eingesetzt werden.

Weitere geeignete azeotrope Schleppmittel offenbaren die US 2004/0242826, die EP-A 778 255, die EP-A 695 736 und der in diesen Schriften zitierte Stand der Technik. Üblicherweise wird die azeotrope Destillation bzw. Rektifikation vorteilhaft bei unterhalb von Atmosphärendruck liegenden Arbeitsdrucken durchgeführt.

Damit umfasst die vorliegende Anmeldung insbesondere ein erfindungsgemäßes Verfahren, bei dem man die Acrylsäure und Glyoxal gemeinsam mit anderen leichter und schwerer als Acrylsäure siedenden Bestandteilen aus dem Produktgasgemisch der heterogen katalysierten partiellen Gasphasenoxidation in eine wässrige flüssige Phase überführt (z. B. durch Absorption mittels einer wässrigen Lösung) und von der dabei resultierenden wässrigen flüssigen Phase mittels azeotroper Rektifikation und/oder Destillation wenigstens eine Teilmenge des Wassers abtrennt, wobei eine flüssige Phase P verbleibt, die nachfolgend erfindungsgemäß kristallisativ behandelt wird. Grundsätzlich kann dabei wie in den Schriften EP-A 1 298 120 sowie EP-A 1 396 484 beschrieben vorgegangen werden, es besteht jedoch keine Notwendigkeit mehr, die in diesen Schriften geforderten besonderen Maßnahme zu ergreifen.

An dieser Stelle sei noch festgehalten, dass bei einer fraktionierenden Kondensation des Produktgasgemischs selbiges anwendungstechnisch zweckmäßig (gegebenenfalls nach zuvor erfolgter direkter und/oder indirekter (z. B. mit einer Quenchflüssigkeit gemäß der EP-A 1 066 239, oder gemäß der EP-A 1 163 201) Kühlung des Produktgasgemischs) in einer trennwirksame Einbauten aufweisenden Trennkolonne in sich selbst aufsteigend unter Seitenabzug einer rohen Acrylsäure (die gegebenenfalls die erfindungsgemäß zu behandelnde flüssige Phase P bildet; gegebenenfalls wird die rohe Acrylsäure zur Erzeugung der flüssigen Phase P noch rektifikativ und/oder destillativ behandelt) fraktionierend kondensiert (vgl. auch EP-A 1 015 410, WO 2004/035514, DE-A 102 43 625, EP-A 1 015 411, DE-A 102 35 847, EP-A 1 159 249, EP-A 1 163 201, EP-A 1 066 239 und EP-A 920 408). Um bei der fraktionierenden Kondensation gegebenenfalls auftretende Acrylsäureverluste zu minimieren, kann der fraktionierenden Kondensation gegebenenfalls zusätzlich eine Absorption mit Wasser und/oder wässriger Lösung überlagert werden.

Eine solchermaßen kondensativ (und gegebenenfalls zusätzlich rektifikativ) erzeugte flüssige Phase wird man dann zweckmäßig einer erfindungsgemäßen kristallisativen Abtrennung unterwerfen, wenn sie sowohl den diesbezüglich erforderlichen Acrylsäuregehalt als auch den auf den Acrylsäuregehalt bezogenen relevanten Gehalt an Glyoxal aufweist.

Dabei gebildetes Glyoxal angereichert enthaltende Mutterlauge kann man dann, wie bereits in dieser Schrift verschiedentlich erwähnt, z. B. gemäß dem Vorbild der EP-A 920 408, oder WO 2004/035514, oder EP-A 1 554 234, oder der Anmeldung PCT/EP2008/050785 bzw. DE-A 102007055086 wenigstens teilweise, vorzugsweise vollständig, in die fraktionierende Kondensation der Acrylsäure aus dem Produktgasgemisch rückführen.

Den Glyoxalauslass wird man dabei unterhalb des Seitenabzugs der rohen Acryläsure ansiedeln.

Die erfindungsgemäße kristallisative Behandlung der flüssigen Phase P, insbesondere einer in vorgenannter Weise kondensativ und/oder absorptiv und/oder rektifikativ gewonnenen flüssigen Phase P, unterliegt grundsätzlich keiner Beschränkung, einschließlich den Verfahren zur Abtrennung der Mutterlauge vom Kristallisat (alle im in dieser Schrift genannten Stand der Technik ausgeführten Verfahren sind anwendbar). D. h., sie kann einstufig, oder mehrstufig, kontinuierlich oder diskontinuierlich durchgeführt werden. Insbesondere kann sie auch als fraktionierende Kristallisation durchgeführt werden. Üblicherweise werden bei einer fraktionierten Kristallisation alle Stufen, die ein Acrylsäurekristallisat erzeugen, das (insbesondere an Glyoxal) reiner als die zugeführte flüssige Phase P ist, Reinigungsstufen genannt und alle anderen Stufen Abtriebsstufen. Zweckmäßig werden mehrstufige Verfahren nach dem Gegenstromprinzip betrieben, bei dem nach der Kristallisation in jeder Stufe das Kristallisat von der Mutterlauge abgetrennt und dieses Kristallisat der jeweiligen Stufe mit dem nächst höheren Reinheitsgrad zugeführt wird, während der Kristallisationsrückstand der jeweiligen Stufe mit dem nächst niedrigeren Reinheitsgrad zugeführt wird.

In der Regel liegt die Temperatur der flüssigen Phase P während des erfindungsgemäßen Verfahrens zwischen -25°C und +14°C, insbesondere zwischen +12°C und -5°C.

Beispielsweise kann das erfindungsgemäße Verfahren als Schichtkristallisation ausgeführt sein (vgl. DE-OS 2606364, EP-A 616998, EP-A 648520 und EP-A 776875). Dabei wird das Kristallisat in Form zusammenhängender, fest anhaftender Schichten ausgefroren. Die Trennung des abgeschiedenen Kristallisats von der verbliebenen Restschmelze (die Mutterlauge) erfolgt durch einfaches Abfließen der Restschmelze. Prinzipiell unterscheidet man zwischen "statischen" und "dynamischen" Schichtkristallisationsverfahren. Kennzeichnend für die dynamische Schichtkristallisation von flüssigen Phasen P ist eine erzwungene Konvektion der flüssigen Phase P. Diese kann durch Umpumpen der flüssigen Phase P durch voll durchströmte Rohre, durch Aufgabe der flüssigen Phase P als Rieselfilm (z.B. gemäß EP-A 616998) oder durch Einleiten von Inertgas in eine flüssige Phase P oder durch Pulsieren erfolgen.

Bei den statischen Verfahren ruht die flüssige Phase P (z.B. in Rohrbündel- oder Plattenwärmeaustauschern) und scheidet sich schichtförmig durch langsame Temperatursenkung auf der Sekundärseite ab. Danach wird die Restschmelze (Mutterlauge) abgelassen, durch langsame Temperaturerhöhung stärker verunreinigte Fraktionen aus der Kristallschicht abgeschwitzt und nachfolgend das Reinprodukt abgeschmolzen (vgl. WO 01/77056).

Erfindungsgemäß bevorzugt wird das erfindungsgemäße Verfahren im Fall aller in dieser Schrift beschriebenen flüssigen Phasen P jedoch gemäß der Lehre der WO 01/77056, der WO 02/055469, der EP-A 1 554 234, der PCT/EP2008/050785, der DE-A 102007055086, der deutschen Anmeldung 102007043759.7, der deutschen Anmeldung 102007043758.9, der deutschen Anmeldung 102007043748.1 und der WO 03/078378 als Suspensionskristallisation ausgeführt.

Dabei wird in der Regel durch Kühlung der flüssigen Phase P eine Acrylsäurekristalle suspendiert enthaltende Kristallisatsuspension erzeugt, wobei die Acrylsäurekristalle einen geringeren und die verbleibende Restschmelze (Mutterlauge) einen höheren Glyoxalgehalt (relativ bezogen auf die jeweilige Gesamtmenge) aufweist, als die zu reinigende flüssige Phase P. Dabei können die Acrylsäurekristalle unmittelbar in Suspension befindlich wachsen und/oder sich als Schicht auf einer gekühlten Wand abscheiden, von der sie anschließend abgekratzt und in der Restschmelze (Mutterlauge) resuspendiert werden.

Alle in der WO 01/77056, WO 02/055469, der EP-A 1 554 234, der PCT/EP2008/050785, der DE-A 102007055086, der deutschen Anmeldung 102007043759.7, der deutschen Anmeldung 102007043758.9, der deutschen Anmeldung 102007043748.1 sowie der WO 03/078378 aufgeführten Suspensionskristaller und Suspensionskristallisationsverfahren kommen erfindungsgemäß in Betracht. In der Regel weist die dabei erzeugte Acrylsäurekristallisatsuspension einen Feststoffgehalt von 20 bis 40 Gew.-% auf.

Weiterhin kommen alle in den vorgenannten (insbesondere die in den vorgenannten WO-)Veröffentlichungen genannten Verfahren zur Trennung von gebildetem Suspensionskristallisat und verbliebener Mutterlauge in Betracht (z.B. mechanische Trennverfahren wie Zentrifugieren). Erfindungsgemäß bevorzugt erfolgt die Trennung in einer Waschkolonne. Bevorzugt handelt es sich dabei um eine Waschkolonne mit erzwungenem Transport der abgeschiedenen Acrylsäurekristalle. Der Kristallisatvolumenanteil im Kristallbett erreicht dabei in der Regel Werte > 0,5. Im Regelfall wird die Waschkolonne bei Werten von 0,6 bis 0,75 betrieben. Als Waschflüssigkeit wird mit Vorteil die Schmelze von in der Waschkolonne vorab gereinigten (abgetrennten) Acrylsäurekristallen verwendet. Die Wäsche erfolgt normalerweise im Gegenstrom. Damit umfasst das erfindungsgemäße Verfahren insbesondere Verfahren, welche folgende Verfahrensschritte umfassen:
a) Auskristallisieren von Acrylsäure aus einer flüssigen Phase P,
b) Trennen des Acrylsäurekristallisats von der verbliebenen Mutterlauge (Restschmelze, flüssigen Restphase),
c) wenigstens teilweises Aufschmelzen des abgetrennten Acrylsäurekristallisats und
d) wenigstens teilweises Rückführen des aufgeschmolzenen Acrylsäurekristallisats zum Schritt b) und/oder zum Schritt a).

Bevorzugt erfolgt dabei der Schritt b) durch Gegenstromwäsche mit in den Schritt b) rückgeführtem aufgeschmolzenem zuvor abgetrenntem Acrylsäurekristallisat.

Insbesondere dann, wenn die Kristallisation als Suspensionskristallisation ausgeführt wird, und noch mehr, wenn die nachfolgende Mutterlaugeabtrennung in einer Waschkolonne ausgeführt wird, und noch mehr, wenn dabei als Waschflüssigkeit die Schmelze von in der Waschkolonne bereits vorab gereinigtem Acrylsäurekristallisat verwendet wird, erweist es sich als günstig, dass die flüssige Phase P häufig Wasser enthält.

D.h., das erfindungsgemäße Verfahren umfasst nicht zuletzt Verfahren, bei denen man die zu reinigende flüssige Phase P unter Einwirkung von Kälte in eine aus Acrylsäurekristallisat und flüssiger Restphase (Restschmelze) bestehende Kristallisatsuspension überführt, wobei der Gewichtsanteil des Acrylsäurekristallisats an Glyoxal kleiner und der Gewichtsanteil der flüssigen Restphase (der Mutterlauge) an Glyoxal größer als der Glyoxalgewichtsanteil der flüssigen Phase P ist, von der Kristallisatsuspension gegebenenfalls einen Teil der verbliebenen Mutterlauge mechanisch abtrennt und das Acrylsäurekristallisat in einer Waschkolonne (vgl. z. B. WO 01/77056, WO 03/041832, WO 03/041833 und WO 98/01414) von verbliebener Mutterlauge mit der Maßgabe befreit, dass
a) die flüssige Phase P bezogen auf die darin enthaltene Acrylsäure 0,20 bis 30, häufig bis 20, oft bis 10 Gew.-% an Wasser enthält, und
b) als Waschflüssigkeit die Schmelze von in der Waschkolonne gereinigtem Acrylsäurekristallisat verwendet wird.

Insbesondere umfasst das erfindungsgemäße Verfahren vorstehende Verfahren, wobei die flüssige Phase P ≥ 90 Gew.-% Acrylsäure, oder ≥ 95 Gew.-% Acrylsäure aufweist.

Weiterhin ist es erfindungsgemäß vorteilhaft, wenn der Wassergehalt der flüssigen Phase P bei vorstehend beschriebenen Verfahrensweisen (bzw. ganz generell bei Anwendung des erfindungsgemäßen Verfahrens), bezogen auf in der flüssigen Phase P enthaltene Acrylsäure, 0,2 bzw. 0,4 bis 8, bzw. bis 10, bzw. bis 20, bzw. bis 30 Gew.-%, bzw. 0,6 bis 5 Gew.-%, bzw. 0,6 bis 3 Gew.-% beträgt.

Selbstredend ist das erfindungsgemäße Verfahren auch auf alle in den in dieser Schrift als Stand der Technik aufgeführten Schriften erwähnten Roh-Acrylsäuren anwendbar, soweit diese den erforderlichen Acrylsäuregehalt und zusätzlich den erforderlichen Glyoxalgehalt enthalten.

Alles Vorgenannte gilt vor allem dann, wenn die Waschkolonne eine Waschkolonne mit erzwungenem Transport der Acrylsäurekristalle ist, und dies vor allem dann, wenn es eine hydraulische oder eine mechanische Waschkolonne z. B. gemäß der WO 01/77056 ist und sie wie dort ausgeführt betrieben wird.

Alles Vorgenannte gilt vor allem dann, wenn die Waschkolonne gemäß den Lehren der WO 03/041832 sowie der WO 03/041833 ausgeführt ist und betrieben wird.

Das erfindungsgemäße Verfahren gestattet so, mit der Sequenz Partialoxidation wenigstens eines C₃-Vorläufers unter Erhalt eines einen erhöhten Glyoxalgehaltes aufweisenden Produktgasgemischs, fraktionierende Acrylsäurekondensation aus dem Produktgasgemisch der Partialoxidation, Suspensionskristallisation des entnommenen Acrylsäurekondensats und Abtrennung des Suspensionskristallisats von verbliebener Mutterlauge in einer Waschkolonne unter Anwendung einer Reinkristallisatschmelze als Waschflüssigkeit, auf effizienteste Art und Weise und unter Anwendung lediglich einer Kristallisationsstufe die Herstellung von an Glyoxal frei zu bezeichnender und somit superabsorbertauglicher Acrylsäure (solche Acrylsäure kann selbstredend auch für alle anderen in der WO 02/055469 und WO 03/078378 angesprochenen Verwendungen eingesetzt werden und dies vor allem dann, wenn von einer billigen, die Nebenproduktbildung von Glyoxal bedingenden C₃-Vorläufer-Rohstoffquelle für die Partialoxidation ausgegangen wird).

Selbstverständlich werden alle in dieser Schrift aufgeführten Verfahrensschritte polymerisationsinhibiert durchgeführt. Dabei kann wie im aufgeführten Stand der Technik beschrieben vorgegangen werden. Eine herausragende Position unter der Gesamtmenge der verfügbaren Acrylsäure-Prozeßstabilisatoren nehmen Dibenzo-1,4-thiazin (PTZ), 4-Hydroxy-2,2,6,6-tetramethylpiperidin-1-oxyl (4-OH-TEMPO) und p-Methoxyphenol (MEHQ) ein, die entweder jeweils für sich, oder paarweise oder als Dreiergemisch Bestandteil der erfindungsgemäß zu behandelnden flüssigen Phase P sein können. Üblicherweise beträgt die Gesamtmenge von an in der flüssigen Phase P enthaltenen Polymerisationsinhibitoren, bezogen auf die Gesamtmenge an darin enthaltener Acrylsäure, 0,001 bis 2 Gew.-%.

Aufgrund unerwünschter Bildung von Acrylsäure-Oligomeren (Michael-Addukte) in der flüssigen Phase P bei deren sich selbst überlassen, wird das erfindungsgemäße Verfahren möglichst umgehend nach Erzeugung der flüssigen Phase P angewendet.

In erfindungsgemäß vorteilhafter Weise werden bei Anwendung des erfindungsgemäβen Verfahrens auch z. B. in der flüssigen Phase P enthaltene C₄- (z.B. Buten-1, Butadien, n-Butan etc.) Partialoxidationsfolgeprodukte wie z.B. Methacrylsäure, Buttersäuren, Butyraldehyde etc. mitabgetrennt. Sie können, bezogen auf die enthaltene molare Acrylsäuremenge, in den gleichen Mengen wie Glyoxal in der flüssigen Phase P (insbesondere in allen in dieser Schrift explizit ausgeführten flüssigen Phasen P) enthalten sein. Das gleiche gilt für Acrolein, Formaldehyd, Acetaldehyd, Propionaldehyd sowie alle C₅- und C₆-Partialoxidationsfolgeprodukte sowie gegebenenfalls in der flüssigen Phase P enthaltene Polyglyoxale und Polyglyoxalhydrate.

Damit umfasst vorliegende Erfindung insbesondere die folgenden Ausführungsformen:
1. Verfahren zur Auftrennung von in einem Produktgasgemisch einer partiellen heterogen katalysierten Gasphasenoxidation einer C₃-Vorläuferverbindung der Acrylsäure als Hauptprodukt enthaltener Acrylsäure und als Nebenprodukt enthaltenem Glyoxal, bei dem man eine flüssige Phase P erzeugt, die zu wenigstens 90 % ihres Gewichtes aus Acrylsäure besteht und, bezogen auf die in ihr enthaltene molare Menge an Acrylsäure, wenigstens 200 mol.ppm Glyoxal enthält, dadurch gekennzeichnet, dass die Trennung des Glyoxals von der Acrylsäure aus der flüssigen Phase P heraus kristallisativ erfolgt, wobei sich die Acrylsäure im gebildeten Kristallisat und das Glyoxal sich in der bei der Kristallisation verbleibenden Mutterlauge anreichert.
2. Verfahren gemäß Ausführungsform 1, dadurch gekennzeichnet, dass die flüssige Phase P, bezogen auf die in ihr enthaltene molare Menge an Acrylsäure, wenigstens 300 mol.ppm Glyoxal enthält.
3. Verfahren gemäß Ausführungsform 1, dadurch gekennzeichnet, dass die flüssige Phase P, bezogen auf die in ihr enthaltene molare Menge an Acrylsäure, wenigstens 400 mol.ppm Glyoxal enthält.
4. Verfahren gemäß Ausführungsform 1, dadurch gekennzeichnet, dass die flüssige Phase P, bezogen auf die in ihr enthaltene molare Menge an Acrylsäure, wenigstens 500 mol.ppm Glyoxal enthält.
5. Verfahren gemäß Ausführungsform 1, dadurch gekennzeichnet, dass die flüssige Phase P, bezogen auf die in ihr enthaltene molare Menge an Acrylsäure, wenigstens 1000 mol.ppm Glyoxal enthält.
6. Verfahren gemäß Ausführungsform 1, dadurch gekennzeichnet, dass die flüssige Phase P, bezogen auf die in ihr enthaltene molare Menge an Acrylsäure, wenigstens 1500 mol.ppm Glyoxal enthält.
7. Verfahren gemäß einer der Ausführungsformen 1 bis 6, dadurch gekennzeichnet, dass die flüssige Phase P zu wenigstens 95 % ihres Gewichtes aus Acrylsäure besteht.
8. Verfahren gemäß einer der Ausführungsformen 1 bis 6, dadurch gekennzeichnet, dass die flüssige Phase P zu wenigstens 96 % ihres Gewichtes aus Acrylsäure besteht.
9. Verfahren gemäß einer der Ausführungsformen 1 bis 6, dadurch gekennzeichnet, dass die flüssige Phase P zu wenigstens 97 % ihres Gewichtes aus Acrylsäure besteht.
10. Verfahren gemäß einer der Ausführungsformen 1 bis 9, dadurch gekennzeichnet, dass die C₃-Vorläuferverbindung Propylen ist.
11. Verfahren gemäß einer der Ausführungsformen 1 bis 9, dadurch gekennzeichnet, dass die C₃-Vorläuferverbindung Acrolein ist.
12. Verfahren gemäß einer der Ausführungsformen 1 bis 9, dadurch gekennzeichnet, dass die C₃-Vorläuferverbindung Propan ist.
13. Verfahren gemäß einer der Ausführungsformen 1 bis 9, dadurch gekennzeichnet, dass die C₃-Vorläuferverbindung Glyzerin ist.
14. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 200 mol.ppm an C₂-Verbindungen enthält.
15. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 300 mol.ppm an C₂-Verbindungen enthält.
16. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 400 mol.ppm an C₂-Verbindungen enthält.
17. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 500 mol.ppm an C₂-Verbindungen enthält.
18. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 750 mol.ppm an C₂-Verbindungen enthält.
19. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasgemisch eingesetzt wird, das, bezogen auf die in ihm enthaltene molare Menge der C₃-Vorläuferverbindung, ≥ 1000 mol.ppm an C₂-Verbindungen enthält.
20. Verfahren gemäß einer der Ausführungsformen 1 bis 13, dadurch gekennzeichnet, dass für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 1500 mol.ppm an C₂-Verbindungen enthält.
21. Verfahren gemäß einer der Ausführungsformen 14 bis 20, dadurch gekennzeichnet, dass das Reaktionsgasausgangsgemisch zu 4 bis 20 Vol.-% C₃-Vorläuferverbindung enthält.
22. Verfahren gemäß einer der Ausführungsformen 14 bis 21, dadurch gekennzeichnet, dass das Reaktionsgasausgangsgemisch zu ≥ 1 Gew.-% Wasserdampf enthält.
23. Verfahren gemäß einer der Ausführungsformen 14 bis 21, dadurch gekennzeichnet, dass das Reaktionsgasausgangsgemisch zu ≥ 2 Gew.-% Wasserdampf enthält.
24. Verfahren gemäß einer der Ausführungsformen 14 bis 21, dadurch gekennzeichnet, dass das Reaktionsgasausgangsgemisch zu ≥ 3 Gew.-% Wasserdampf enthält.
25. Verfahren gemäß einer der Ausführungsformen 14 bis 21, dadurch gekennzeichnet, dass das Reaktionsgasausgangsgemisch zu ≥ 5 Gew.-% Wasserdampf enthält.
26. Verfahren gemäß einer der Ausführungsformen 14 bis 21, dadurch gekennzeichnet, dass das Reaktionsgasausgangsgemisch zu ≥ 7 Gew.-% Wasserdampf enthält.
27. Verfahren gemäß einer der Ausführungsformen 1 bis 26, dadurch gekennzeichnet, dass die flüssige Phase P aus dem Produktgasgemisch der partiellen heterogen katalysierten Gasphasenoxidation durch Anwendung wenigstens eines nicht kristallisativen thermischen Trennverfahrens erzeugt wurde.
28. Verfahren gemäß Ausführungsform 27, dadurch gekennzeichnet, dass das wenigstens eine nicht kristallisative thermische Trennverfahren wenigstens ein Trennverfahren aus der Gruppe enthaltend Absorption, partielle Kondensation, fraktionierende Kondensation, Rektifikation, Strippung und Desorption umfasst.
29. Verfahren gemäß Ausführungsform 27 oder 28, dadurch gekennzeichnet, dass bei der Kristallisation verbleibende, Glyoxal angereichert enthaltende Mutterlauge, in wenigstens eines der nicht kristallisativen thermischen Trennverfahren rückgeführt wird.
30. Verfahren gemäß Ausführungsform 29, dadurch gekennzeichnet, dass bei der Kristallisation verbleibende, Glyoxal angereichert enthaltende Mutterlauge, in eine fraktionierende Kondensation des Produktgasgemischs der heterogen katalysierten Gasphasenoxidation rückgeführt wird.
31. Verfahren gemäß einer der Ausführungsformen 1 bis 30, dadurch gekennzeichnet, dass die kristallisative Trennung mittels einer Suspensionskristallisation vorgenommen wird.
32. Verfahren gemäß Ausführungsform 31, dadurch gekennzeichnet, dass bei der Suspensionskristallisation gebildetes Suspensionskristallisat und verbliebene Mutterlauge mittels einer Waschkolonne voneinander getrennt werden.
33. Verfahren gemäß Ausführungsform 32, dadurch gekennzeichnet, dass in der Waschkolonne das Suspensionskristallisat mit der Schmelze von in der Waschkolonne vorab abgetrennten Acrylsäurekristallen gewaschen wird.
34. Verfahren gemäß einer der Ausführungsformen 1 bis 33, dadurch gekennzeichnet, dass es die folgenden Verfahrensschritte umfasst:
   a. Auskristallisieren von Acrylsäure aus der flüssigen Phase P;
   b. Trennen des Acrylsäurekristallisats von beim Auskristallisieren verbleibender Mutterlauge;
   c. wenigstens teilweises Aufschmelzen des in Schritt b) abgetrennten Acrylsäurekristallisats;
   d. wenigstens teilweises Rückführen des aufgeschmolzenen Acrylsäurekristallisats aus Schritt c) zum Schritt b) und/oder Schritt a).
35. Verfahren gemäß einer der Ausführungsformen 1 bis 34, dadurch gekennzeichnet, dass die flüssige Phase P, bezogen auf die in ihr enthaltene Acrylsäure, 0,2 bis 30 Gew.-% Wasser enthält.
36. Verfahren gemäß einer der Ausführungsformen 1 bis 35, dadurch gekennzeichnet, dass man zur Erzeugung der flüssigen Phase P im Produktgasgemisch enthaltene Acrylsäure und enthaltenes Glyoxal in eine wässrige flüssige Phase überführt, und von dieser flüssigen wässrigen Phase mittels azeotroper Rektifikation wenigstens eine Teilmenge des Wassers abtrennt, wobei die flüssige Phase P verbleibt.
37. Verfahren gemäß einer der Ausführungsformen 1 bis 36, dadurch gekennzeichnet, dass die flüssige Phase P das Glyoxal zu wenigstens 50 mol-% als monomeres Glyoxal-Monohydrat und/oder monomeres Glyoxal-Dihydrat enthält.
38. Verfahren gemäß einer der Ausführungsformen 1 bis 36, dadurch gekennzeichnet, dass die flüssige Phase P das Glyoxal zu wenigstens 70 mol-% als monomeres Glyoxal-Monohydrat und/oder monomeres Glyoxal-Dihydrat enthält.
39. Verfahren gemäß einer der Ausführungsformen 1 bis 36, dadurch gekennzeichnet, dass die flüssige Phase P das Glyoxal zu wenigstens 90 mol-% als monomeres Glyoxal-Monohydrat und/oder monomeres Glyoxal-Dihydrat enthält.
40. Verfahren nach einem der Ansprüche 1 bis 39, dadurch gekennzeichnet, dass das Produktgasgemisch, bezogen auf die in ihm enthaltene molare Menge an Acrylsäure, wenigstens 200 mol.ppm Glyoxal enthält.
41. Verfahren nach einem der Ansprüche 1 bis 39, dadurch gekennzeichnet, dass das Produktgasgemisch, bezogen auf die in ihm enthaltene molare Menge an Acrylsäure, wenigstens 400 mol.ppm Glyoxal enthält.
42. Verfahren nach einem der Ansprüche 1 bis 39, dadurch gekennzeichnet, dass das Produktgasgemisch, bezogen auf die in ihm enthaltene molare Menge an Acrylsäure, wenigstens 750 mol.ppm Glyoxal enthält.
43. Verfahren nach einem der Ansprüche 1 bis 42, dadurch gekennzeichnet, dass im Rahmen der Erzeugung der flüssigen Phase P im Produktgasgemisch enthaltene Acrylsäure in die kondensierte (z.B. flüssige) Phase überführt und dabei gasförmig verbleibendes Restgas wenigstens Teilweise in die partielle heterogen katalysierte Gasphasenoxidation der C₃-Vorläuferverbindung rückgeführt wird.
44. Verfahren gemäß einer der Ausführungsformen 1 bis 43, dadurch gekennzeichnet, dass sich ein Verfahren anschließt, bei dem Acrylsäurekristallisat aufgeschmolzen und in wenigstens ein Polymerisat radikalisch einpolymerisiert wird.

### Beispiele:

I. Nachweis der die unerwünschte radikalische Polymerisation von Acrylsäure in überdurchschnittlicher Weise fördernden Wirkung von monomerem Glyoxal in Acrylsäure
1. Herstellung einer Lösung von monomerem Glyoxal in wasserfreiem Methanol
2,0 g feinteiliges Glyoxaltrimer-Dihydrat (von der Firma Fluka, Reinheit > 95 %) und 5,0 g feinteiliges P₂O₅ (von der Firma Aldrich, Reinheit > 98 %) wurden homogen vermischt. Anschließend wurde das Gemisch bei einem Druck < 50 mbar auf 180 °C erhitzt, und der sich dabei kontinuierlich entwickelnde gasförmige Strom aus monomerem Glyoxal und gegebenenfalls geringen Spuren an Wasserdampf über feinteiliges P₂O₅ (bindet die Restspuren an Wasserdampf) in wasserfreies Methanol (von der Firma Aldrich, Reinheit > 99,8%) geleitet.
Bezogen auf die Ausgangsmenge an Methanol wurden 0,3 Gew.-% an monomerem Glyoxal eingeleitet und im Methanol gelöst (vgl. auch Y. Chen, L. Zhu in "Wavelength-Dependent Photolysis of Glyoxal in the 290-420 nm Region", J. Phys. Chem. A, 2003, 107, 4643-4651). Die resultierende methanolische Lösung wird im Folgenden als Lösung M bezeichnet (in der methanolischen Lösung bilden sich aus dem Glyoxal und Methanol die entsprechenden thermisch labilen Acetale, die bei mäßig erhöhter Temperatur wieder in monomeres Glyoxal und Methanol rückspalten).
2. Herstellung von mit geringen Mengen verschiedener möglicher Nebenproduktaldehyde dotierten Reinacrylsäuren
Es wurden 5 nicht unterscheidbare Proben (von jeweils 0,5 ml) an Reinacrylsäure zubereitet. Dazu wurde mit dem Monomethylether des Hydrochinons (MEHQ) lagerstabilisierte Reinacrylsäure unter vermindertem Druck frisch überdestilliert und mit 10 gew.ppm Phenotiazin (PTZ) stabilisiert.
Die Reinheit der so erzeugten Reinacrylsäureproben betrug > 99,8 Gew.-% bei einem Aldehyd- und Ketongesamtgehalt < 10 gew.ppm. Die Aufbewahrung der Proben erfolgte im gefrorenen Zustand.
Anschließend wurden die Proben durch Zuwiegen der entsprechenden Aldehyde wie folgt dotiert (im Fall des Glyoxal wurde dazu die Lösung M verwendet; die mol.ppm beziehen sich stets auf die enthaltene molare Acrylsäuremenge; wegen des Methanolgehaltes der Lösung M wurde bei den Dotierungen mit von Glyoxal verschiedenen Aldehyden eine entsprechende Methanolmenge zudotiert):

| | | |
|---|---|---|
| Probe 1: | 86 mol.ppm | monomeres Glyoxal |
| Probe 2: | 96 mol.ppm | Benzaldehyd |
| Probe 3: | 166 mol.ppm | Formaldehyd |
| Probe 4: | 104 mol.ppm | 2-Fufural |
| Probe 5: | 113 mol.ppm | Acetaldehyd |

Unter Luftatmosphäre in identische Glasampullen mit einem Fassungsvermögen von jeweils 1,8 ml eingeschmolzen wurden die Ampullen jeweils unmittelbar nach ihrer Fertigstellung bei einer Temperatur von 120 °C im Umlufttrockenschrank drehend gelagert, um vollständige Durchmischung zu gewährleisten. Dann wurde die Zeit T bis zur vollständigen Polymerisation der jeweiligen Probe visuell erfasst.
Die Versuchsreihe wurde dreimal wiederholt und die Messwerte arithmetisch gemittelt. Die mittleren Ergebnisse für die Zeit T der jeweiligen Proben betrugen:

| | |
|---|---|
| Probe 1: | 117 min |
| Probe 2: | 222 min |
| Probe 3: | 197 min |
| Probe 4: | 199 min |
| Probe 5: | 174 min |

Die erhaltenen Ergebnisse weisen die Ausnahmestellung von monomerem Glyoxal aus.
II. Erfindungsgemäße kristallisative Auftrennung
1. In eine an Glyoxal freie Acrylsäure mit den nachfolgenden Verunreinigungsgehalten (die Angaben beziehen sich auf gaschromatographische Analyse; die Wasserbestimmung erfolgte nach Karl-Fischer, die PTZ-Analyse nasschemisch)

| | |
|---|---|
| 37 | gew.ppm Allylacrylat, |
| 3319 | gew.ppm Benzaldehyd, |
| 3404 | gew.ppm Diacrylsäure, |
| 1,94 | gew.-% Essigsäure, |
| 0,91 | gew.-% Propionsäure, |
| 4211 | gew.ppm Furfural-2 (Furan-2-aldehyd), |
| 33 | gew.ppm Furfural-3 (Furan-3-aldehyd), |
| 348 | gew.ppm Wasser, und |
| 297 | gew.ppm Phenothiazin, |

und einem Acrylsäuregehalt von 95,80 Gew.-% wurde ein wie in I.1. erzeugter gasförmiger Strom an monomerem Glyoxal eingeleitet, bis sie, bezogen auf die enthaltene Acrylsäuremenge, mit 1741 mol.ppm an monomerem Glyoxal dotiert war (= 1277 gew.ppm bezogen auf das Gesamtgewicht der verunreinigten Acrylsäure).
Anschließend wurde sie in einem Mengenstrom von 135 kg/h und einer Temperatur von 17,3 °C einem Kühlscheibenkristallisator (mit einem Flüssigkeitsfassungsvolumen von ca. 95 Liter) mit gewischten Kühlscheiben zugeführt. Der Kristallisator wies 7 kreisförmige, im äquidistanten Abstand von 12 cm in einem Trog hintereinander hängend angeordnete, Kühlscheiben auf. Der Durchmesser der Kühlscheiben betrug 32 cm und die Scheibendicke lag, bei einer Wanddicke von 2,5 mm, bei 15 mm. Die Kühlflächen waren aus Edelstahl (DIN-Werkstoff 1.4571) gefertigt. Die Kühlscheiben wurden von einer Wasser-Glykol-Mischung als Kühlmittel (353 I/h) durchströmt (55 Vol.-% Wasser, 45 Vol.-% Glykol). Das Kühlmittel strömte mit einer Eingangstemperatur von 2,5 °C in die erste Kühlscheibe und verließ mit einer Ausgangstemperatur von 8,6 °C die letzte Kühlscheibe. Die dotierte Acrylsäure und das Kühlmittel wurden im Gegenstrom durch den Kristallisator geführt. Die ungewischten Ränder der Kühlscheiben wurden mit einem um diese umlaufenden Hohlprofil (schlauchförmiges Rohr mit einem Außendurchmesser von 12 mm) begleitbeheizt, um eine Verkrustung mit Kristallisat zu vermeiden. Zu diesem Zweck wurden die Hohlprofile der Kühlscheiben parallel mit derselben, jedoch eine Eintrittstemperatur von 24 °C aufweisenden, Wasser-Glykol-Mischung parallel in einer Gesamtmenge von 51 l/h durchströmt. Die Wischer der Kühlplatten wurden mittels einer horizontalen Welle mit einer Drehzahl von 26 U/min angetrieben. Die im Kristallisator erzeugte Kristallisatsuspension verließ denselben mit einer Temperatur von 10,6 °C.
Ein Mengenstrom von 119 kg/h Acrylsäurekristallisatsuspension wurde dem Kristallisator über ein Grobsieb (Maschenweite ca. 3 mm; das Grobsieb verfolgte den Zweck, im Kristallisator gebildete größere Kristallisatagglomerate zurückzuhalten, da diese ein Verstopfen der Suspensionszuführleitung zur Waschkolonne bedingen könnten) mit der Temperatur von 10,6 °C entnommen und von oben in eine hydraulische Waschkolonne gepumpt. Diese bestand aus einem kreiszylindrischen Doppelglasmantel (Höhe: 1000 mm; Außendurchmesser des inneren Zylinders: 100 mm, Wanddicke des inneren Zylinders: 9 mm; der äußere Zylinder war um den inneren Zylinder herum mit Hilfe von entsprechenden Dichtungen abgedichtet; der Zwischenraum war mit Luft gefüllt; Außendurchmesser des äuβeren Zylinders: 130 mm; Wanddicke des äußeren Zylinders: 5 mm). Im inneren Zylinder zentriert eingehängt war ein metallisches Filterrohr (aus Edelstahl (DIN-Werkstoff 1.4571)), das einen Außendurchmesser von 20 mm und eine Wanddicke von 1,6 mm aufwies. Mit seiner Unterkante 200 mm oberhalb des unteren Endes des Glasmantels befand sich ein 40 mm hohes, einen Außendurchmesser von 20 mm aufweisendes, zylindrisch umlaufendes Filter ins Filterrohr eingepasst, über das die in der Acrylsäurekristallisatsuspension enthaltene Flüssigphase abgeführt wurde. Durch Abfuhr der vorgenannten Flüssigphase wurde im Glaszylinder um das Filterrohr herum ein Kristallisatbett erzeugt, dessen Bettlänge (Höhe) mit Hilfe einer optischen Höhenmessung bei einem Wert von 510 mm (gemessen vom unteren Ende des Glasmantels aus) gehalten wurde. Zum Einregeln der Betthöhe wurde ein Steuerstrom von etwa 95 kg/h über das Filterrohr abgeführter Flüssigphase am Kopf der Waschkolonne in selbige rückgeführt. Am unteren Ende des Glaszylinders wurde das gewaschene Kristallisatbett mit einer rotierenden Messerscheibe (30 U/min) abgetragen und das abgetragene Kristallisat in einem Schmelzkreis bestehend aus Pumpe, Heizer, Inhibitorzudosierung und entsprechenden Rohrleitungen umgepumpt und aufgeschmolzen. Die Inhibitorzudosierung erfolgte in Form von in Reinschmelze gelöstem PTZ so, dass der PTZ-Gehalt des Schmelzkreises ca. 200 gew.ppm betrug. Die Temperatur im Schmelzkreis vor der zur Kristallisatwäsche erforderlichen Reinschmelzerückführung in das untere Ende der Waschkolonne betrug 18 °C.
Nur etwa 5,5 kg/h aufgeschmolzenes Kristallisat wurden dem Schmelzkreis als gereinigtes Produkt (Reinschmelze) entnommen. Der Mengenstrom an dem Schmelzkreis entnommenem Reinprodukt wurde dabei so geregelt, dass sich im Kristallisatbett, von außen sichtbar auf einer Höhe von etwa 90 bis 110 mm oberhalb des unteren Endes des Glaszylinders, eine Waschfront ausbildete. Zur Regelung des Mengenstroms an dem Schmelzkreis entnommener Reinschmelze wurde die Temperatur im Kristallisatbett auf einer Höhe von 100 mm oberhalb des unteren Endes des Glaszylinders herangezogen, die durch das Ansteuern eines Ventils in der Reinschmelzeentnahmeleitung (Anpassung des Entnahmestroms) auf einen Wert von 11,5 °C eingeregelt wurde.
Die Analyse der aus dem Filterrohr herausgeführten Flüssigphase (Mutterlauge) ergab einen Glyoxalgehalt von 1365 gew.ppm (bezogen auf das Gewicht der Mutterlauge).
Die Analyse des aus dem Schmelzkreis entnommenen, aufgeschmolzenen Kristallisats ergab einen Glyoxalgehalt von weniger als 0,1 gew.ppm (bezogen auf das Kristallisatgewicht).
Aus beiden vorgenannten Werten errechnet sich somit ein Abreicherungskoeffizient von A^{Gly} > 13650.
2. Der Versuch II.1. wurde wiederholt, die Glyoxal-Dotierung wurde jedoch mit einer von der Fa. Aldrich bezogenen und bei 25 °C gelagerten wässrigen Lösung vorgenommen, die gemäß eigener Analyse zu 40 % ihres Gewichts Glyoxal enthielt (gerechnet als monomeres Glyoxal). Mit der Dotierung der Acrylsäure ging eine geringfügige Niederschlagsbildung einher, die vermutlich auf höhermolekulare, in der Acrylsäure schwerlösliche Polyglyoxale (bzw. Hydrate derselben) zurückzuführen ist. Es wurde daher abfiltriert und als Filtrat eine mit 1877 mol.ppm Glyoxal dotierte Acrylsäure erhalten. Mit dieser wurde wie in Versuch II.1. verfahren. Der so experimentell ermittelte Abreicherungskoeffizient A^{Gly} (gerechnet als monomeres Glyoxal) war > 14720.US Provisional Patent Applications No. 61/084109, eingereicht am 28. Juli 2008 und No. 61/091900, eingereicht am 26. August 2008, sind in die vorliegende Anmeldung durch Literaturhinweis eingefügt. Im Hinblick auf die oben genannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Verfahren zur Auftrennung von in einem Produktgasgemisch einer partiellen heterogen katalysierten Gasphasenoxidation einer C₃-Vorläuferverbindung der Acrylsäure als Hauptprodukt enthaltener Acrylsäure und als Nebenprodukt enthaltenem Glyoxal, bei dem man eine flüssige Phase P erzeugt, die zu wenigstens 90 % ihres Gewichtes aus Acrylsäure besteht und, bezogen auf die in ihr enthaltene molare Menge an Acrylsäure, wenigstens 200 mol.ppm Glyoxal enthält, **dadurch gekennzeichnet, dass** die Trennung des Glyoxals von der Acrylsäure aus der flüssigen Phase P heraus kristallisativ erfolgt, wobei sich die Acrylsäure im gebildeten Kristallisat und das Glyoxal sich in der bei der Kristallisation verbleibenden Mutterlauge anreichert.

2. Verfahren nach Anspruch 1, **dadurch gekennzeichnet, dass** die flüssige Phase P, bezogen auf die in ihr enthaltene molare Menge an Acrylsäure, wenigstens 300 mol.ppm Glyoxal enthält.

3. Verfahren nach Anspruch 1, **dadurch gekennzeichnet, dass** die flüssige Phase P, bezogen auf die in ihr enthaltene molare Menge an Acrylsäure, wenigstens 400 mol.ppm Glyoxal enthält.

4. Verfahren nach Anspruch 1, **dadurch gekennzeichnet, dass** die flüssige Phase P, bezogen auf die in ihr enthaltene molare Menge an Acrylsäure, wenigstens 500 mol.ppm Glyoxal enthält.

5. Verfahren nach Anspruch 1, **dadurch gekennzeichnet, dass** die flüssige Phase P, bezogen auf die in ihr enthaltene molare Menge an Acrylsäure, wenigstens 1000 mol.ppm Glyoxal enthält.

6. Verfahren nach Anspruch 1, **dadurch gekennzeichnet, dass** die flüssige Phase P, bezogen auf die in ihr enthaltene molare Menge an Acrylsäure, wenigstens 1500 mol.ppm Glyoxal enthält.

7. Verfahren nach einem der Ansprüche 1 bis 6, **dadurch gekennzeichnet, dass** die flüssige Phase P zu wenigstens 95 % ihres Gewichtes aus Acrylsäure besteht.

8. Verfahren nach einem der Ansprüche 1 bis 6, **dadurch gekennzeichnet, dass** die flüssige Phase P zu wenigstens 96 % ihres Gewichtes aus Acrylsäure besteht.

9. Verfahren nach einem der Ansprüche 1 bis 6, **dadurch gekennzeichnet, dass** die flüssige Phase P zu wenigstens 97 % ihres Gewichtes aus Acrylsäure besteht.

10. Verfahren nach einem der Ansprüche 1 bis 9, **dadurch gekennzeichnet, dass** die C₃-Vorläuferverbindung Propylen ist.

11. Verfahren nach einem der Ansprüche 1 bis 9, **dadurch gekennzeichnet, dass** die C₃-Vorläuferverbindung Acrolein ist.

12. Verfahren nach einem der Ansprüche 1 bis 9, **dadurch gekennzeichnet, dass** die C₃-Vorläuferverbindung Propan ist.

13. Verfahren nach einem der Ansprüche 1 bis 9, **dadurch gekennzeichnet, dass** die C₃-Vorläuferverbindung Glyzerin ist.

14. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 200 mol.ppm an C₂-Verbindungen enthält.

15. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 300 mol.ppm an C₂-Verbindungen enthält.

16. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 400 mol.ppm an C₂-Verbindungen enthält.

17. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 500 mol.ppm an C₂-Verbindungen enthält.

18. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, > 750 mol.ppm an C₂-Verbindungen enthält.

19. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 1000 mol.ppm an C₂-Verbindungen enthält.

20. Verfahren nach einem der Ansprüche 1 bis 13, **dadurch gekennzeichnet, dass** für die partielle heterogen katalysierte Gasphasenoxidation einer C₃-Vorläuferverbindung ein Reaktionsgasausgangsgemisch eingesetzt wird, das, bezogen auf die in ihm enthaltene molare Menge der C₃-Vorläuferverbindung, ≥ 1500 mol.ppm an C₂-Verbindungen enthält.

21. Verfahren nach einem der Ansprüche 14 bis 20, **dadurch gekennzeichnet, dass** das Reaktionsgasausgangsgemisch zu 4 bis 20 Vol.-% C₃-Vorläuferverbindung enthält.

22. Verfahren nach einem der Ansprüche 14 bis 21, **dadurch gekennzeichnet, dass** das Reaktionsgasausgangsgemisch zu ≥ 1 Gew.-% Wasserdampf enthält.

23. Verfahren nach einem der Ansprüche 14 bis 21, **dadurch gekennzeichnet, dass** das Reaktionsgasausgangsgemisch zu ≥ 2 Gew.-% Wasserdampf enthält.

24. Verfahren nach einem der Ansprüche 14 bis 21, **dadurch gekennzeichnet, dass** das Reaktionsgasausgangsgemisch zu ≥ 3 Gew.-% Wasserdampf enthält.

25. Verfahren nach einem der Ansprüche 14 bis 21, **dadurch gekennzeichnet, dass** das Reaktionsgasausgangsgemisch zu ≥ 5 Gew.-% Wasserdampf enthält.

26. Verfahren nach einem der Ansprüche 14 bis 21, **dadurch gekennzeichnet, dass** das Reaktionsgasausgangsgemisch zu ≥ 7 Gew.-% Wasserdampf enthält.

27. Verfahren nach einem der Ansprüche 1 bis 26, **dadurch gekennzeichnet, dass** die flüssige Phase P aus dem Produktgasgemisch der partiellen heterogen katalysierten Gasphasenoxidation durch Anwendung wenigstens eines nicht kristallisativen thermischen Trennverfahrens erzeugt wurde.

28. Verfahren nach Anspruch 27, **dadurch gekennzeichnet, dass** das wenigstens eine nicht kristallisative thermische Trennverfahren wenigstens ein Trennverfahren aus der Gruppe enthaltend Absorption, partielle Kondensation, fraktionierende Kondensation, Rektifikation, Strippung und Desorption umfasst.

29. Verfahren nach Anspruch 27oder 28, **dadurch gekennzeichnet, dass** bei der Kristallisation verbleibende, Glyoxal angereichert enthaltende Mutterlauge, in wenigstens eines der nicht kristallisativen thermischen Trennverfahren rückgeführt wird.

30. Verfahren nach Anspruch 29, **dadurch gekennzeichnet, dass** bei der Kristallisation verbleibende, Glyoxal angereichert enthaltende Mutterlauge, in eine fraktionierende Kondensation des Produktgasgemischs der heterogen katalysierten Gasphasenoxidation rückgeführt wird.

31. Verfahren nach einem der Ansprüche 1 bis 30, **dadurch gekennzeichnet, dass** die kristallisative Trennung mittels einer Suspensionskristallisation vorgenommen wird.

32. Verfahren nach Anspruch 31, **dadurch gekennzeichnet, dass** bei der Suspensionskristallisation gebildetes Suspensionskristallisat und verbliebene Mutterlauge mittels einer Waschkolonne voneinander getrennt werden.

33. Verfahren nach Anspruch 32, **dadurch gekennzeichnet, dass** in der Waschkolonne das Suspensionskristallisat mit der Schmelze von in der Waschkolonne vorab abgetrennten Acrylsäurekristallen gewaschen wird.

34. Verfahren nach einem der Ansprüche 1 bis 33, **dadurch gekennzeichnet, dass** es die folgenden Verfahrensschritte umfasst:
a) Auskristallisieren von Acrylsäure aus der flüssigen Phase P;
b) Trennen des Acrylsäurekristallisats von beim Auskristallisieren verbleibender Mutterlauge;
c) wenigstens teilweises Aufschmelzen des in Schritt b) abgetrennten Acrylsäurekristallisats;
d) wenigstens teilweises Rückführen des aufgeschmolzenen Acrylsäurekristallisats aus Schritt c) zum Schritt b) und/oder Schritt a).

35. Verfahren nach einem der Ansprüche 1 bis 34, **dadurch gekennzeichnet, dass** die flüssige Phase P, bezogen auf die in ihr enthaltene Acrylsäure, 0,2 bis 30 Gew.-% Wasser enthält.

36. Verfahren nach einem der Ansprüche 1 bis 35, **dadurch gekennzeichnet, dass** man zur Erzeugung der flüssigen Phase P im Produktgasgemisch enthaltene Acrylsäure und enthaltenes Glyoxal in eine wässrige flüssige Phase überführt, und von dieser flüssigen wässrigen Phase mittels azeotroper Rektifikation wenigstens eine Teilmenge des Wassers abtrennt, wobei die flüssige Phase P verbleibt.

37. Verfahren nach einem der Ansprüche 1 bis 36, **dadurch gekennzeichnet, dass** die flüssige Phase P das Glyoxal zu mehr als 50 mol-% als monomeres Glyoxal-Monohydrat und/oder monomeres Glyoxal-Dihydrat enthält.

38. Verfahren nach einem der Ansprüche 1 bis 36, **dadurch gekennzeichnet, dass** die flüssige Phase P das Glyoxal zu mehr als 70 mol-% als monomeres Glyoxal-Monohydrat und/oder monomeres Glyoxal-Dihydrat enthält.

39. Verfahren nach einem der Ansprüche 1 bis 36, **dadurch gekennzeichnet, dass** die flüssige Phase P das Glyoxal zu mehr als 90 mol-% als monomeres Glyoxal-Monohydrat und/oder monomeres Glyoxal-Dihydrat enthält.

40. Verfahren nach einem der Ansprüche 1 bis 39, **dadurch gekennzeichnet, dass** das Produktgasgemisch, bezogen auf die in ihm enthaltene molare Menge an Acrylsäure, wenigstens 200 mol.ppm Glyoxal enthält.

41. Verfahren nach einem der Ansprüche 1 bis 39, **dadurch gekennzeichnet, dass** das Produktgasgemisch, bezogen auf die in ihm enthaltene molare Menge an Acrylsäure, wenigstens 400 mol.ppm Glyoxal enthält.

42. Verfahren nach einem der Ansprüche 1 bis 39, **dadurch gekennzeichnet, dass** das Produktgasgemisch, bezogen auf die in ihm enthaltene molare Menge an Acrylsäure, wenigstens 750 mol.ppm Glyoxal enthält.

43. Verfahren nach einem der Ansprüche 1 bis 42, **dadurch gekennzeichnet, dass** im Rahmen der Erzeugung der flüssigen Phase P im Produktgasgemisch enthaltene Acrylsäure in die kondensierte Phase überführt und dabei gasförmig verbleibendes Restgas wenigstens Teilweise in die partielle heterogen katalysierte Gasphasenoxidation der C₃-Vorläuferverbindung rückgeführt wird.

44. Verfahren nach einem der Ansprüche 1 bis 43, **dadurch gekennzeichnet, dass** sich ein Verfahren anschließt, bei dem Acrylsäurekristallisat aufgeschmolzen und in wenigstens ein Polymerisat radikalisch einpolymerisiert wird

## Claims

1. A process for separating acrylic acid present as a main product and glyoxal present as a by-product in a product gas mixture of a partial heterogeneously catalyzed gas phase oxidation of a C₃ precursor compound of acrylic acid, in which a liquid phase P is obtained which consists of acrylic acid to an extent of at least 90% of its weight and, based on the molar amount of acrylic acid present therein, comprises at least 200 molar ppm of glyoxal, which comprises separating the glyoxal from the acrylic acid in the liquid phase P by crystallization, the acrylic acid being enriched in the crystals formed and the glyoxal in the mother liquor which remains in the course of crystallization.

2. The process according to claim 1, wherein the liquid phase P, based on the molar amount of acrylic acid present therein, comprises at least 300 molar ppm of glyoxal.

3. The process according to claim 1, wherein the liquid phase P, based on the molar amount of acrylic acid present therein, comprises at least 400 molar ppm of glyoxal.

4. The process according to claim 1, wherein the liquid phase P, based on the molar amount of acrylic acid present therein, comprises at least 500 molar ppm of glyoxal.

5. The process according to claim 1, wherein the liquid phase P, based on the molar amount of acrylic acid present therein, comprises at least 1000 molar ppm of glyoxal.

6. The process according to claim 1, wherein the liquid phase P, based on the molar amount of acrylic acid present therein, comprises at least 1500 molar ppm of glyoxal.

7. The process according to any one of claims 1 to 6, wherein the liquid phase P consists of acrylic acid to an extent of at least 95% of its weight.

8. The process according to any one of claims 1 to 6, wherein the liquid phase P consists of acrylic acid to an extent of at least 96% of its weight.

9. The process according to any one of claims 1 to 6, wherein the liquid phase P consists of acrylic acid to an extent of at least 97% of its weight.

10. The process according to any one of claims 1 to 9, wherein the C₃ precursor compound is propylene.

11. The process according to any one of claims 1 to 9, wherein the C₃ precursor compound is acrolein.

12. The process according to any one of claims 1 to 9, wherein the C₃ precursor compound is propane.

13. The process according to any one of claims 1 to 9, wherein the C₃ precursor compound is glycerol.

14. The process according to any one of claims 1 to 13, wherein a starting reaction gas mixture which, based on the molar amount of the C₃ precursor compound present therein, comprises > 200 molar ppm of C₂ compounds is used for the partial heterogeneously catalyzed gas phase oxidation of a C₃ precursor compound.

15. The process according to any one of claims 1 to 13, wherein a starting reaction gas mixture which, based on the molar amount of the C₃ precursor compound present therein, comprises > 300 molar ppm of C₂ compounds is used for the partial heterogeneously catalyzed gas phase oxidation of a C₃ precursor compound.

16. The process according to any one of claims 1 to 13, wherein a starting reaction gas mixture which, based on the molar amount of the C₃ precursor compound present therein, comprises > 400 molar ppm of C₂ compounds is used for the partial heterogeneously catalyzed gas phase oxidation of a C₃ precursor compound.

17. The process according to any one of claims 1 to 13, wherein a starting reaction gas mixture which, based on the molar amount of the C₃ precursor compound present therein, comprises > 500 molar ppm of C₂ compounds is used for the partial heterogeneously catalyzed gas phase oxidation of a C₃ precursor compound.

18. The process according to any one of claims 1 to 13, wherein a starting reaction gas mixture which, based on the molar amount of the C₃ precursor compound present therein, comprises > 750 molar ppm of C₂ compounds is used for the partial heterogeneously catalyzed gas phase oxidation of a C₃ precursor compound.

19. The process according to any one of claims 1 to 13, wherein a starting reaction gas mixture which, based on the molar amount of the C₃ precursor compound present therein, comprises > 1000 molar ppm of C₂ compounds is used for the partial heterogeneously catalyzed gas phase oxidation of a C₃ precursor compound.

20. The process according to any one of claims 1 to 13, wherein a starting reaction gas mixture which, based on the molar amount of the C₃ precursor compound present therein, comprises > 1500 molar ppm of C₂ compounds is used for the partial heterogeneously catalyzed gas phase oxidation of a C₃ precursor compound.

21. The process according to any one of claims 14 to 20, wherein the starting reaction gas mixture comprises from 4 to 20% by volume of C₃ precursor compound.

22. The process according to any one of claims 14 to 21, wherein the starting reaction gas mixture comprises > 1% by weight of water vapor.

23. The process according to any one of claims 14 to 21, wherein the starting reaction gas mixture comprises > 2% by weight of water vapor.

24. The process according to any one of claims 14 to 21, wherein the starting reaction gas mixture comprises ≥ 3% by weight of water vapor.

25. The process according to any one of claims 14 to 21, wherein the starting reaction gas mixture comprises ≥ 5% by weight of water vapor.

26. The process according to any one of claims 14 to 21, wherein the starting reaction gas mixture comprises > 7% by weight of water vapor.

27. The process according to any one of claims 1 to 26, wherein the liquid phase P has been obtained from the product gas mixture of the partial heterogeneously catalyzed gas phase oxidation by employing at least one noncrystallizative thermal separation process.

28. The process according to claim 27, wherein the at least one noncrystallizative thermal separation process comprises at least one separation process from the group comprising absorption, partial condensation, fractional condensation, rectification, stripping and desorption.

29. The process according to claim 27 or 28, wherein mother liquor which comprises enriched glyoxal and remains in the course of crystallization is recycled into at least one of the noncrystallizative thermal separation processes.

30. The process according to claim 29, wherein mother liquor which comprises enriched glyoxal and remains in the course of crystallization is recycled into a fractional condensation of the product gas mixture of the heterogeneously catalyzed gas phase oxidation.

31. The process according to any one of claims 1 to 30, wherein the crystallizative separation is undertaken by means of a suspension crystallization.

32. The process according to claim 31, wherein suspension crystals formed in the course of suspension crystallization and remaining mother liquor are separated from one another by means of a wash column.

33. The process according to claim 32, wherein the suspension crystals are washed in the wash column with the melt of acrylic acid crystals removed beforehand in the wash column.

34. The process according to any one of claims 1 to 33, which comprises the following process steps :
a) crystallizing acrylic acid out of the liquid phase P;
b) separating the acrylic acid crystals from mother liquor which remains in the course of crystallization;
c) at least partly melting the acrylic acid crystals removed in step b);
d) at least partly recycling the molten acrylic acid crystals from step c) to step b) and/or step a).

35. The process according to any one of claims 1 to 34, wherein the liquid phase P, based on acrylic acid present therein, comprises from 0.2 to 30% by weight of water.

36. The process according to any one of claims 1 to 35, wherein the liquid phase P is obtained by transferring acrylic acid present and glyoxal present in the product gas mixture to an aqueous liquid phase, and removing at least a portion of the water from this liquid aqueous phase by means of azeotropic rectification, which leaves the liquid phase P.

37. The process according to any one of claims 1 to 36, wherein the liquid phase P comprises the glyoxal in the form of monomeric glyoxal monohydrate and/or monomeric glyoxal dihydrate to an extent of more than 50 mol%.

38. The process according to any one of claims 1 to 36, wherein the liquid phase P comprises the glyoxal in the form of monomeric glyoxal monohydrate and/or monomeric glyoxal dihydrate to an extent of more than 70 mol%.

39. The process according to any one of claims 1 to 36, wherein the liquid phase P comprises the glyoxal in the form of monomeric glyoxal monohydrate and/or monomeric glyoxal dihydrate to an extent of more than 90 mol%.

40. The process according to any one of claims 1 to 39, wherein the product gas mixture, based on the molar amount of acrylic acid present therein, comprises at least 200 molar ppm of glyoxal.

41. The process according to any one of claims 1 to 39, wherein the product gas mixture, based on the molar amount of acrylic acid present therein, comprises at least 400 molar ppm of glyoxal.

42. The process according to any one of claims 1 to 39, wherein the product gas mixture, based on the molar amount of acrylic acid present therein, comprises at least 750 molar ppm of glyoxal.

43. The process according to any one of claims 1 to 42, wherein, in the course of obtaining the liquid phase P, acrylic acid present in the product gas mixture is transferred to the condensed phase while residual gas remaining in gaseous form is recycled at least partly into the partial heterogeneously catalyzed gas phase oxidation of the C₃ precursor compound.

44. The process according to any one of claims 1 to 43, which is followed by a process in which acrylic acid crystals are melted and free-radically polymerized into at least one polymer.

## Revendications

1. Procédé de séparation d'acide acrylique contenu en tant que produit principal et de glyoxal contenu en tant que produit secondaire dans un mélange gazeux de produits d'une oxydation partielle en phase gazeuse sous catalyse hétérogène d'un composé précurseur en C₃ de l'acide acrylique, selon lequel une phase liquide P est formée, qui est constituée à au moins 90 % de son poids d'acide acrylique et qui contient, par rapport à la quantité molaire d'acide acrylique qu'elle contient, au moins 200 ppm en moles de glyoxal, **caractérisé en ce que** la séparation du glyoxal de l'acide acrylique dans la phase liquide P a lieu par cristallisation, l'acide acrylique s'accumulant dans le cristallisat formé et le glyoxal dans la liqueur mère restant lors de la cristallisation.

2. Procédé selon la revendication 1, **caractérisé en ce que** la phase liquide P contient, par rapport à la quantité molaire d'acide acrylique qu'elle contient, au moins 300 ppm en moles de glyoxal.

3. Procédé selon la revendication 1, **caractérisé en ce que** la phase liquide P contient, par rapport à la quantité molaire d'acide acrylique qu'elle contient, au moins 400 ppm en moles de glyoxal.

4. Procédé selon la revendication 1, **caractérisé en ce que** la phase liquide P contient, par rapport à la quantité molaire d'acide acrylique qu'elle contient, au moins 500 ppm en moles de glyoxal.

5. Procédé selon la revendication 1, **caractérisé en ce que** la phase liquide P contient, par rapport à la quantité molaire d'acide acrylique qu'elle contient, au moins 1 000 ppm en moles de glyoxal.

6. Procédé selon la revendication 1, **caractérisé en ce que** la phase liquide P contient, par rapport à la quantité molaire d'acide acrylique qu'elle contient, au moins 1 500 ppm en moles de glyoxal.

7. Procédé selon l'une quelconque des revendications 1 à 6, **caractérisé en ce que** la phase liquide P est constituée à au moins 95 % de son poids d'acide acrylique.

8. Procédé selon l'une quelconque des revendications 1 à 6, **caractérisé en ce que** la phase liquide P est constituée à au moins 96 % de son poids d'acide acrylique.

9. Procédé selon l'une quelconque des revendications 1 à 6, **caractérisé en ce que** la phase liquide P est constituée à au moins 97 % de son poids d'acide acrylique.

10. Procédé selon l'une quelconque des revendications 1 à 9, **caractérisé en ce que** le composé précurseur en C₃ est le propylène.

11. Procédé selon l'une quelconque des revendications 1 à 9, **caractérisé en ce que** le composé précurseur en C₃ est l'acroléine.

12. Procédé selon l'une quelconque des revendications 1 à 9, **caractérisé en ce que** le composé précurseur en C₃ est propane.

13. Procédé selon l'une quelconque des revendications 1 à 9, **caractérisé en ce que** le composé précurseur en C₃ est la glycérine.

14. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, ≥ 200 ppm en moles de composés en C₂.

15. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, ≥ 300 ppm en moles de composés en C₂.

16. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, 400 ppm en moles de composés en C₂.

17. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, 500 ppm en moles de composés en C₂.

18. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, 750 ppm en moles de composés en C₂.

19. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, ≥ 1 000 ppm en moles de composés en C₂.

20. Procédé selon l'une quelconque des revendications 1 à 13, **caractérisé en ce qu'**on utilise, pour l'oxydation partielle en phase gazeuse sous catalyse hétérogène d'un composé précurseur en C₃, un mélange réactionnel gazeux initial qui contient, par rapport à la quantité molaire du composé précurseur en C₃ qu'il contient, 1 500 ppm en moles de composés en C₂.

21. Procédé selon l'une quelconque des revendications 14 à 20, **caractérisé en ce que** le mélange réactionnel gazeux initial contient de 4 à 20 % en volume de composé précurseur en C₃.

22. Procédé selon l'une quelconque des revendications 14 à 21, **caractérisé en ce que** le mélange réactionnel gazeux initial contient ≥ 1 % en poids de vapeur d'eau.

23. Procédé selon l'une quelconque des revendications 14 à 21, **caractérisé en ce que** le mélange réactionnel gazeux initial contient ≥ 2 % en poids de vapeur d'eau.

24. Procédé selon l'une quelconque des revendications 14 à 21, **caractérisé en ce que** le mélange réactionnel gazeux initial contient ≥ 3 % en poids de vapeur d'eau.

25. Procédé selon l'une quelconque des revendications 14 à 21, **caractérisé en ce que** le mélange réactionnel gazeux initial contient ≥ 5 % en poids de vapeur d'eau.

26. Procédé selon l'une quelconque des revendications 14 à 21, **caractérisé en ce que** le mélange réactionnel gazeux initial contient ≥ 7 % en poids de vapeur d'eau.

27. Procédé selon l'une quelconque des revendications 1 à 26, **caractérisé en ce que** la phase liquide P a été formée à partir du mélange gazeux de produits de l'oxydation partielle en phase gazeuse sous catalyse hétérogène par application d'au moins un procédé de séparation thermique non cristallisant.

28. Procédé selon la revendication 27, **caractérisé en ce que** ledit au moins un procédé de séparation thermique non cristallisant comprend au moins un procédé de séparation du groupe contenant l'absorption, la condensation partielle, la condensation fractionnée, la rectification, l'extraction et la désorption.

29. Procédé selon la revendication 27 ou 28, **caractérisé en ce que** liqueur mère contenant le glyoxal accumulé restant lors de la cristallisation est recyclée dans au moins un des procédés de séparation thermiques non cristallisants.

30. Procédé selon la revendication 29, **caractérisé en ce que** liqueur mère contenant le glyoxal accumulé restant lors de la cristallisation est recyclée dans une condensation fractionnée du mélange gazeux de produits de l'oxydation en phase gazeuse sous catalyse hétérogène.

31. Procédé selon l'une quelconque des revendications 1 à 30, **caractérisé en ce que** la séparation par cristallisation est réalisée au moyen d'une cristallisation en suspension.

32. Procédé selon la revendication 31, **caractérisé en ce que** le cristallisat en suspension formé lors de la cristallisation en suspension et la liqueur mère restante sont séparés l'un de l'autre au moyen d'une colonne de lavage.

33. Procédé selon la revendication 32, **caractérisé en ce que** le cristallisat en suspension est lavé dans la colonne de lavage avec la masse fondue de cristaux d'acide acrylique séparés auparavant dans la colonne de lavage.

34. Procédé selon l'une quelconque des revendications 1 à 33, **caractérisé en ce qu'**il comprend les étapes de procédé suivantes :
a) la cristallisation d'acide acrylique à partir de la phase liquide P ;
b) la séparation du cristallisat d'acide acrylique de la liqueur mère restant lors de la cristallisation ;
c) la fusion au moins partielle du cristallisat d'acide acrylique séparé à l'étape b) ;
d) le recyclage au moins partiel du cristallisat d'acide acrylique fondu de l'étape c) dans l'étape b) et/ou l'étape a).

35. Procédé selon l'une quelconque des revendications 1 à 34, **caractérisé en ce que** la phase liquide P contient, par rapport à l'acide acrylique qu'elle contient, 0,2 à 30 % en poids d'eau.

36. Procédé selon l'une quelconque des revendications 1 à 35, **caractérisé en ce que**, pour la formation de la phase liquide P, l'acide acrylique et le glyoxal contenus dans le mélange gazeux de produits sont transférés dans une phase liquide aqueuse, et au moins une partie de l'eau de cette phase liquide aqueuse est séparée par rectification azéotropique, la phase liquide P restant.

37. Procédé selon l'une quelconque des revendications 1 à 36, **caractérisé en ce que** la phase liquide P contient le glyoxal à hauteur de plus de 50 % en moles en tant que glyoxal monohydraté monomère et/ou de glyoxal dihydraté monomère.

38. Procédé selon l'une quelconque des revendications 1 à 36, **caractérisé en ce que** la phase liquide P contient le glyoxal à hauteur de plus de 70 % en moles en tant que glyoxal monohydraté monomère et/ou de glyoxal dihydraté monomère.

39. Procédé selon l'une quelconque des revendications 1 à 36, **caractérisé en ce que** la phase liquide P contient le glyoxal à hauteur de plus de 90 % en moles en tant que glyoxal monohydraté monomère et/ou de glyoxal dihydraté monomère.

40. Procédé selon l'une quelconque des revendications 1 à 39, **caractérisé en ce que** le mélange gazeux de produits contient, par rapport à la quantité molaire d'acide acrylique qu'il contient, au moins 200 ppm en moles de glyoxal.

41. Procédé selon l'une quelconque des revendications 1 à 39, **caractérisé en ce que** le mélange gazeux de produits contient, par rapport à la quantité molaire d'acide acrylique qu'il contient, au moins 400 ppm en moles de glyoxal.

42. Procédé selon l'une quelconque des revendications 1 à 39, **caractérisé en ce que** le mélange gazeux de produits contient, par rapport à la quantité molaire d'acide acrylique qu'il contient, au moins 750 ppm en moles de glyoxal.

43. Procédé selon l'une quelconque des revendications 1 à 42, **caractérisé en ce que** l'acide acrylique contenu dans le mélange gazeux de produits est transféré en phase condensée dans le cadre de la formation de la phase liquide P, et le gaz résiduel restant est au moins partiellement recyclé dans l'oxydation partielle en phase gazeuse sous catalyse hétérogène du composé précurseur en C₃.

44. Procédé selon l'une quelconque des revendications 1 à 43, **caractérisé en ce qu'**il est suivi par un procédé lors duquel le cristallisat d'acide acrylique est fondu et polymérisé radicalairement dans au moins un polymère.
